(19) Europäisches Patentamt
European Patent Office
Office européen des brevets

(11) EP 2 307 365 B1

(12) **EUROPEAN PATENT SPECIFICATION**

(45) Date of publication and mention of the grant of the patent:
**27.03.2013 Bulletin 2013/13**

(21) Application number: **09770902.6**

(22) Date of filing: **24.06.2009**

(51) Int Cl.:
***C07D 209/46*** (2006.01) ***C07D 209/48*** (2006.01)
***C07D 231/56*** (2006.01) ***C07D 401/04*** (2006.01)
***C07D 487/04*** (2006.01)

(86) International application number:
**PCT/US2009/048368**

(87) International publication number:
**WO 2009/158369 (30.12.2009 Gazette 2009/53)**

(54) **SYNTHESIS AND USE OF HETEROCYCLIC ANTIBACTERIAL AGENTS**

SYNTHESE UND VERWENDUNG HETEROCYCLISCHER ANTIBAKTERIELLER MITTEL

SYNTHÈSE ET UTILISATION D AGENTS ANTIBACTÉRIENS HÉTÉROCYCLIQUES

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **25.06.2008 US 75383**

(43) Date of publication of application:
**13.04.2011 Bulletin 2011/15**

(73) Proprietor: **Merck Sharp & Dohme Corp.**
**Rahway, NJ 07065-0907 (US)**

(72) Inventors:
- **REDDY, Panduranga, Adulla P.**
**Walpole**
**Massachusetts 02081 (US)**
- **MANSOOR, Umar, Faruk**
**Framingham**
**Massachusetts 01702 (US)**
- **SIDDIQUI, M. Arshad**
**Newton**
**Massachusetts 02464 (US)**

(74) Representative: **Horgan, James Michael Frederic**
**Merck Sharp & Dohme Limited**
**Hertford Road**
**Hoddesdon, Herts. EN11 9BU (GB)**

(56) References cited:
**WO-A-99/41246**
**WO-A-99/41276**
**WO-A-2008/027466**
**WO-A-2008/154642**
**JP-A- 2002 088 046**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 307 365 B1

## Description

## FIELD OF THE INVENTION

This invention relates generally to heterocycles that can inhibit UDP-3-O-(R-3-hydroxymyristoyl)-N-acetylglucosamine deacetylase (LpxC), and as a result have antimicrobial activity.

## BACKGROUND OF THE INVENTION

Lipid A is the hydrophobic anchor of lipopolysaccharide (LPS) and forms the major lipid component of the outer monolayer of the outer membrane of gram-negative bacteria. Lipid A is required for bacterial growth and inhibition of its biosynthesis is lethal to the bacteria. Furthermore, blocking Lipid A biosynthesis increases the sensitivity of bacteria to other antibiotics.

One of the key enzymes of bacterial lipid A biosynthesis is LpxC. LpxC catalyzes the removal of the N-acetyl group of UDP-3-O-(R-3-hydroxymyristoyl)-N-acetylglucosamine. The LpxC enzyme is essential in gram negative bacteria for the biosynthesis of Lipid A, and it is notably absent from mammalian genomes. Since LpxC is essential for Lipid A biosynthesis and inhibition of Lipid A biosynthesis is lethal to bacteria, inhibitors of LpxC have utility as antibiotics. In addition, the absence of LpxC from mammalian genomes reduces potential toxicity of LpxC inhibitors in mammals. Accordingly, LpxC is an attractive target for antibacterial drug discovery.

U.S. Patent 5,925,659 teaches that certain heterocyclic hydroxamate compounds, in particular oxazoline compounds, have the ability to inhibit LpxC.

WO2004/00744 refers to N-Hydroxyamide derivatives having LpxC inhibitory activity and thus possessing antibacterial activity.

WO2004/062601 also refers to small molecule inhibitors of LpxC.

WO2007/064732 refers to N-Hydroxyamide derivatives having LpxC inhibitory activity and thus possessing antibacterial activity.

WO2008/027466 also refers to small molecule inhibitors of LpxC.

JP-A-2002088046 (Japan Science and Tech Corp) discloses

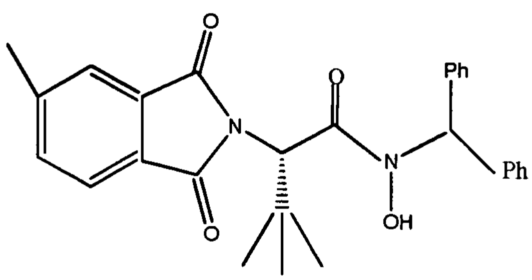

WO-A-9941276 (Molecumetics Limited) discloses

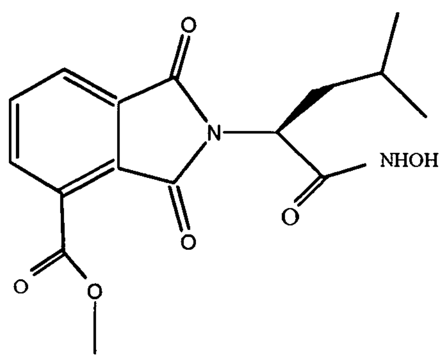

as a β-sheet mimetic and a protease inhibitor (of metalloproteinases leucine aminopeptidase M and thermolysin).

WO-A-9941246 (Du Pont Pharmaceuticals Company) discloses cyclic sulfonamide derivatives as metalloproteinase inhibitors.

There is a need in the art for small molecule inhibitors of LpxC as potential antibacterial agents.

## SUMMARY OF THE INVENTION

In one aspect, the present invention provides compounds of Formula (I):

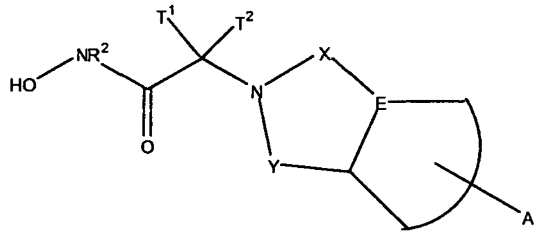

Formula (I)

and pharmaceutically acceptable salts, solvates and esters thereof,
wherein:

$T^1$ is H and $T^2$ is ethyl or isopropyl, wherein said ethyl or isopropyl is substituted with -OH or $NH_2$;
X is C(O);
Y is C(O) or $CH_2$;
E is C, CH, or N;

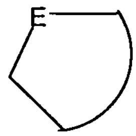

is a six-membered ring selected from the group consisting of aryl, cycloalkenyl, cycloalkyl, heteroaryl, heterocyclenyl and heterocyclyl;

A is selected from the group consisting of ethynyl, phenyl, phenylmethyl, piperidine, piperazine, bromo and chloro, wherein said ethynyl, phenyl, phenylmethyl, piperidine, and piperazine can be unsubstituted or substituted with an additional phenyl or additional ethynyl: wherein said additional phenyl can be unsubstituted or substituted with another phenyl or ethynyl, still further wherein, said another phenyl, can be unsubstituted or substituted with still another phenyl and; wherein said additional ethynyl is substituted with another phenyl,
wherein said another phenyl can be unsubstituted or substituted with still another phenyl;
or A is

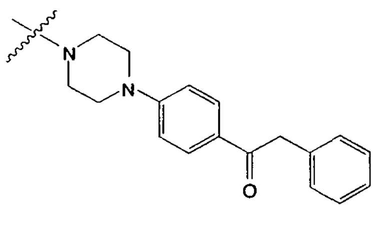;

$R^2$ is hydrogen;

In another aspect, the invention provides the compounds of formula (I) as inhibitors of LpxC, methods of preparing such compounds, pharmaceutical compositions comprising one or more such compounds, methods of preparing pharmaceutical formulations comprising one or more such compounds, and discloses methods of treatment, prevention, inhibition or amelioration of one or more diseases associated with LpxC, using such compounds or pharmaceutical compositions.

In another aspect, disclosed is a method of treating microbial infections.

In a further aspect, the invention provides compositions comprising one or more compounds of Formula (I), an

additional therapeutic agent for treating LpxC receptor mediated disease, and a pharmaceutical carrier.

## DETAILED DESCRIPTION OF THE INVENTION

In its several embodiments, the present invention provides compounds of formula (I) which are a novel class of inhibitors of LpxC, pharmaceutical compositions containing one or more of the compounds, methods of preparing pharmaceutical formulations comprising one or more such compounds, and discloses methods of treatment, prevention or amelioration of microbial infections.

from the group consisting of halo, -OH, O-aryl, O-cycloalkenyl, -O-cycloalkyl, -O-heteroaryl, -O-heterocyclenyl, -O-heterocyclyl, -O-alkyl, -O-alkenyl, -O-alkynyl, -O-aralkyl, -O-aralkenyl, -O-cycloalkenylalkyl, -O-cycloalkylalkyl, -SH, S-aryl, S-cycloalkenyl, -S-cycloalkyl, -S-heteroaryl, -S-heterocyclenyl, -S-heterocyclyl, -S-alkyl, - S-alkenyl, -S-alkynyl, -S-aralkyl, -S-aralkenyl, -S-cycloalkenylalkyl, and -S-cycloalkylalkyl, $-NH_2$, -NH-aryl, -NH-cycloalkenyl, -NH-cycloalkyl, -NH-heteroaryl,-NH-heterocydenyl, -NH-heterocyclyl, -NH-alkyl, -NH-alkenyl, -NH-alkynyl, -NH-aralkyl, -NH-aralkenyl, -NH-cydenylalkyl, -NH-cycloalkylalkyl, aryl, cycloalkenyl, cycloalkyl, heteroaryl, heterocyclenyl, heterocyclyl, alkyl, alkenyl, alkynyl, wherein each of said O-aryl, O-cycloalkenyl, -O-cycloalkyl, -O-heteroaryl, -O-heterocyclenyl, -O-heterocyclyl, -O-alkyl, -O-alkenyl, -O-alkynyl, -O-aralkyl, -O-aralkenyl, -O-cycloalkenylalkyl, -O-cycloalkylalkyl, S-aryl, S-cycloalkenyl, -S-cycloalkyl, -S-heteroaryl, -S-heterocyclenyl, - S-heterocyclyl, -S-alkyl, -S-alkenyl, -S-alkynyl, -S-aralkyl, -S-aralkenyl, -S-cycloalkenylalkyl, -S-cycloalkylalkyl, -NH-aryl, -NH-cycloalkenyl, -NH-cycloalkyl, -NH-heteroaryl, -NH-heterocyclenyl, -NH-heterocyclyl, -NH-alkyl, -NH-alkenyl, -NH-alkynyl, -NH-aralkyl, -NH-aralkenyl, -NH-cycloalkenylalkyl, -NH-cycloalkylalkyl, aryl, cycloalkenyl, cycloalkyl, heteroaryl, heterocyclenyl, heterocyclyl, alkyl, alkenyl and alkynyl can be unsubstituted or substituted with one or more moieties independently selected from the group consisting of halo, -OH, O-aryl, O-cycloalkenyl, -0-cycloalkyl, -O-heteroaryl, -O-heterocyclenyl, -O-heterocyclyl, -O-alkyl, -O-alkenyl, -O-alkynyl, -O-aralkyl, -O-aralkenyl, -O-cycloalkenylalkyl, -O-cycloalkylalkyl, -SH, S-aryl, S-cycloalkenyl, -S-cycloalkyl, -S-heteroaryl, -S-heterocyclenyl, -S-heterocyclyl, -S-alkyl, - S-alkenyl, -S-alkynyl, -S-aralkyl, -S-aralkenyl, -S-cycloalkenylalkyl, -S-cycloalkylalkyl, - NH-aryl, -NH-cycloalkenyl, -NH-cycloalkyl, -NH-heteroaryl, -NH-heterocyclenyl, -NH-heterocyclyl, -NH-alkyl, -NH-alkenyl, -NH-alkynyl, -NH-aralkyl, -NH-aralkenyl, -NH-cycloalkenylalkyl, -NH-cycloalkylalkyl, aryl, cycloalkenyl, cycloalkyl, heteroaryl, heterocyclenyl, heterocyclyl, alkyl, alkenyl and alkynyl;

$R^2$ is independently selected from the group consisting of H, alkyl, alkenyl, alkynyl, cycloalkyl, cycloalkenyl, aryl, heteroaryl, heterocyclyl and heterocyclenyl, wherein each of said alkyl, alkenyl, alkynyl, cycloalkyl, cycloalkenyl, aryl, heteroaryl, heterocyclyl, and heterocyclenyl can be unsubstituted or substituted with up to three moieties independently selected from the group consisting of halogen, alkyl, alkenyl, alkynyl, aryl, cycloalkenyl, cycloalkyl, aralkyl, aralkenyl, cycloalkenylalkyl, cycloalkylalkyl, -OH, O-aryl, O-cycloalkenyl, -O-cycloalkyl, -O-heteroaryl, -O-heterocyclenyl, -O-heterocyclyl, -O-alkyl, -O-alkenyl, -O-alkynyl, -O-aralkyl, -O-aralkenyl, -O-cycloalkenylalkyl, -O-cycloalkylalkyl, -SH, S-aryl, S-cycloalkenyl, -S-cycloalkyl, -S-heteroaryl, -S-heterocyclenyl, -S-heterocyclyl, -S-alkyl, -S-alkenyl, -S-alkynyl, -S-aralkyl, -S-aralkenyl, -S-cycloalkenylalkyl, -S-cycloalkylalkyl, $-NH_2$, -NH-aryl, -NH-cycloalkenyl, -NH-cycloalkyl, -NH-heteroaryl, -NH-heterocyclenyl, -NH-heterocyclyl, -NH-alkyl, -NH-alkenyl, -NH-alkynyl, -NH-aralkyl, -NH-aralkenyl, -NH-cycloalkenylalkyl, and -NH-cycloalkylalkyl.

The compound according to claim 1, wherein $T^1$ and $T^2$ are each independently selected from the group consisting of H and $(C_1-C_8)$alkyl, wherein said $(C_1-C_8)$alkyl, can be unsubstituted or substituted with one or more moieties independently selected from the group consisting of alkyl, -OH, $NH_2$.

In another aspect, the invention provides a novel class of compounds as inhibitors of LpxC, methods of preparing such compounds, pharmaceutical compositions comprising one or more such compounds, methods of preparing pharmaceutical formulations comprising one or more such compounds, and methods of treatment, prevention, inhibition or amelioration of one or more diseases associated with LpxC, using such compounds or pharmaceutical compositions.

In another aspect, the invention provides a method of treating microbial infections.

In a further aspect, the invention provides compositions comprising one or more compounds of Formula (I), an additional therapeutic agent for treating LpxC receptor mediated disease, and a pharmaceutical carrier.

## DETAILED DESCRIPTION OF THE INVENTION

In its several embodiments, the present invention provides a novel class of inhibitors of LpxC, pharmaceutical compositions containing one or more of the compounds, methods of preparing pharmaceutical formulations comprising one or more such compounds, and methods of treatment, prevention or amelioration of microbial infections.

In one embodiment, the present invention provides compounds, which are represented by structural Formula (I):

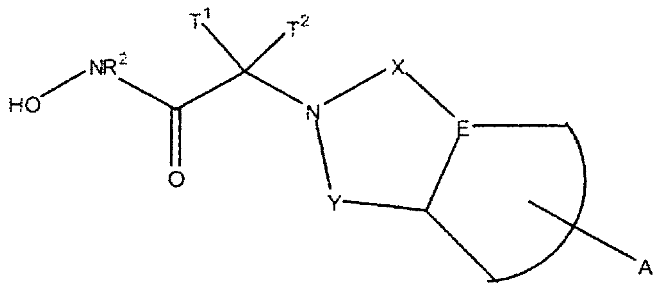

(I)

In one embodiment,

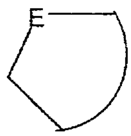

is phenyl, wherein E is C or CH.

In another embodiment,

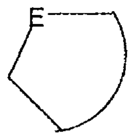

is piperazine, wherein E is N.

In one embodiment, A is alkynyl, wherein said alkynyl is substituted with phenyl, wherein said phenyl can be unsubstituted or substituted with an additional phenyl.

In another embodiment, A is ethynyl, wherein said ethynyl is substituted with phenyl, wherein said phenyl can be unsubstituted or substituted with an additional phenyl.

In still another embodiment, A is ethynyl, wherein said ethynyl is substituted with phenyl, wherein said phenyl can be unsubstituted or para substituted with an additional phenyl.

In yet another embodiment, A is ethynyl, wherein said ethynyl is substituted with phenyl, wherein said phenyl can be unsubstituted or meta substituted with an additional phenyl.

In one embodiment, A is phenyl, wherein said phenyl can be unsubstituted or substituted with alkynyl, wherein said alkynyl is substituted with phenyl.

In another embodiment, A is phenyl, wherein said phenyl can be unsubstituted or substituted with ethynyl, wherein said ethynyl is substituted with an additional phenyl.

In yet another embodiment, A is phenyl, wherein said phenyl can be unsubstituted or substituted with an additional phenyl.

In still another embodiment, A is phenyl, wherein said phenyl can be unsubstituted or para substituted with an additional phenyl.

In one embodiment, A is aralkyl, wherein said aralkyl is substituted with alkynyl, wherein said alkynyl is substituted with aryl.

In another embodiment, A is phenylalkyl, wherein said phenylalkyl is substituted with alkynyl, wherein said alkynyl is substituted with aryl.

In yet another embodiment, A is phenylmethyl, wherein said phenylmethyl is substituted with alkynyl, wherein said alkynyl is substituted with aryl.

In still another embodiment, A is phenylmethyl, wherein said phenylmethyl is substituted with ethynyl, wherein said ethynyl is substituted with aryl.

In another embodiment, A is phenylmethyl, wherein said phenylmethyl is substituted with ethynyl, wherein said ethynyl is substituted with phenyl.

In still another embodiment, A is phenylmethyl, wherein said phenylmethyl is para substituted with ethynyl,

wherein said ethynyl is substituted with phenyl.

In yet another embodiment, A is phenylmethyl, wherein said phenylmethyl is para substituted with phenyl.

In one embodiment, A is piperidinyl, wherein said piperidinyl is substituted with phenyl, wherein said phenyl is substituted with an additional phenyl.

In another embodiment, A is piperidinyl, wherein said piperidinyl is para substituted with phenyl, wherein said phenyl is substituted with an additional phenyl.

In still another embodiment, A is piperidinyl, wherein said piperidinyl is para substituted with phenyl, wherein said phenyl is para substituted with an additional phenyl.

In yet another embodiment, A is piperidinyl, wherein said piperidinyl is substituted with phenyl, wherein said phenyl is substituted with alkynyl, wherein said alkynyl is substituted with an additional phenyl.

In another embodiment, A is piperidinyl, wherein said piperidinyl is para substituted with phenyl, wherein said phenyl is substituted with alkynyl, wherein said alkynyl is substituted with phenyl.

In still another embodiment, A is piperidinyl, wherein said piperidinyl is para substituted with phenyl, wherein said phenyl is para substituted with alkynyl, wherein said alkynyl is substituted with an additional phenyl.

In yet another embodiment, A is piperidinyl, wherein said piperidinyl is para substituted with phenyl, wherein said phenyl is para substituted with ethynyl, wherein said ethynyl is substituted with an additional phenyl.

In one embodiment, A is piperazinyl, wherein said piperazinyl is substituted with phenyl.

In another embodiment, A is piperazinyl, wherein said piperazinyl is para substituted with phenyl.

In still another embodiment, A is piperazinyl, wherein said piperazinyl is substituted with phenyl, further wherein said phenyl is substituted with alkynyl, wherein said alkynyl is substituted with phenyl.

In yet another embodiment, A is piperazinyl, wherein said piperazinyl is substituted with phenyl, wherein said phenyl is substituted with alkynyl, wherein said alkynyl is substituted with an additional phenyl.

In another embodiment, A is piperazinyl, wherein said piperazinyl is substituted with phenyl, wherein said phenyl is substituted with ethynyl, wherein said ethynyl is substituted with an additional phenyl.

In still another embodiment, A is piperazinyl, wherein said piperazinyl is para substituted with phenyl, wherein said phenyl is substituted with ethynyl, wherein said ethynyl is substituted with an additional phenyl.

In yet another embodiment, A is piperazinyl, wherein said piperazinyl is substituted with phenyl, wherein said phenyl is substituted with an additional phenyl.

In another embodiment, A is piperazinyl, wherein said piperazinyl is para substituted with phenyl, wherein said phenyl is substituted with an additional phenyl.

In still another embodiment, A is piperazinyl, wherein said piperazinyl is para substituted with phenyl, wherein said phenyl is para substituted with an additional phenyl.

In still another embodiment, A is piperazinyl, wherein said piperazinyl is para substituted with phenyl, wherein said phenyl is para substituted with aralkylcarbonyl.

In yet another embodiment, A is piperazinyl, wherein said piperazinyl is para substituted with phenyl, further wherein said phenyl is para substituted with phenylmethylcarbonyl.

In one embodiment, A is halo.

In another embodiment, A is bromo.

In yet another embodiment, A is chloro.

In one embodiment, the Compounds of Formula (I) have the Formulae (IA, IB, IC, ID, IE, IIA, IIB, IIC, IID, and IIE):

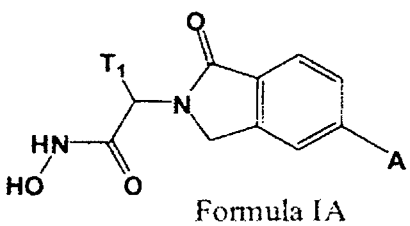

Formula IA ,

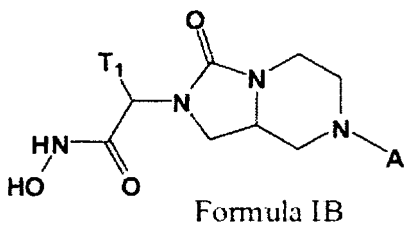

Formula IB .

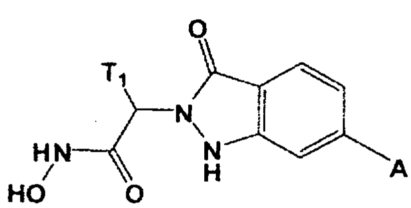
Formula IC ,

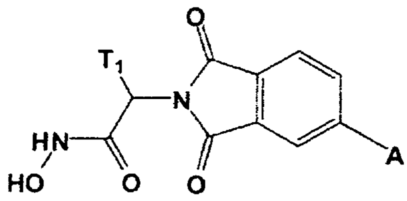
Formula ID ,

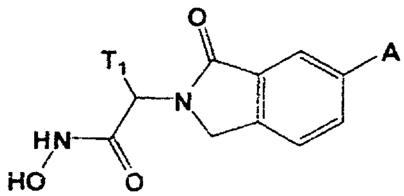
Formula IE ,

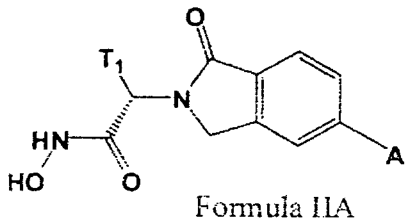
Formula IIA ,

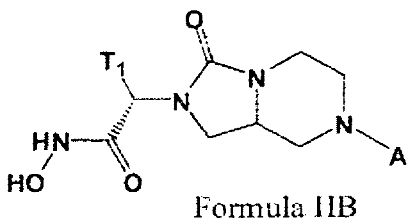
Formula IIB ,

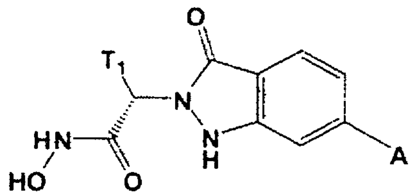
Formula IIC ,

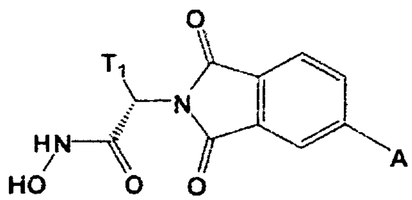
Formula IID ,

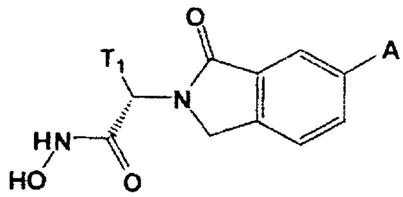
Formula IIE

wherein $T^1$ and A are as defined above for the Compounds of Formula (I).

In one embodiment, $T^1$ is

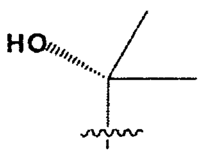
,
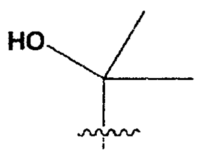
,
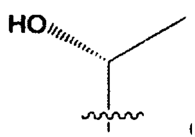
,
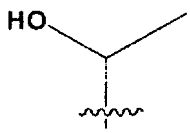
.

In another embodiment, $T^1$ is

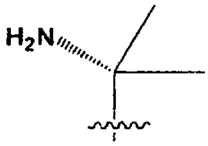,
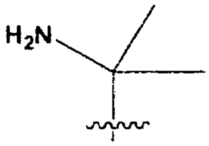
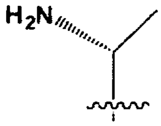

or

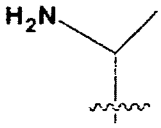.

In one embodiment, A is

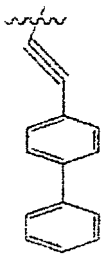.

In another embodiment, A is

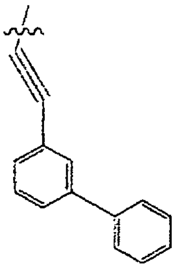.

In one embodiment, A is

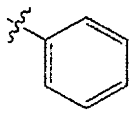.

In another embodiment, A is

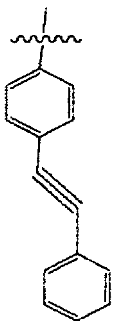.

In still another embodiment, A is

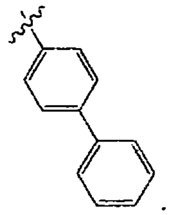

In one embodiment, A is

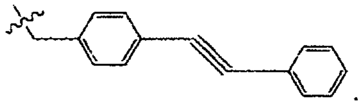

in another embodiment, A is

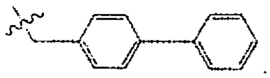

In one embodiment, A is

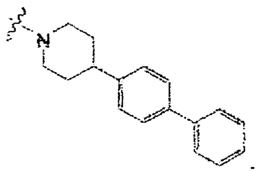

In one embodiment, A is

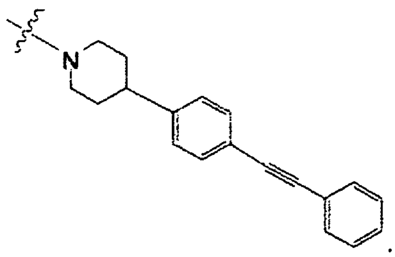

In another embodiment, A is

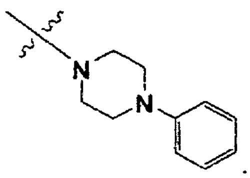

In still another embodiment, A is

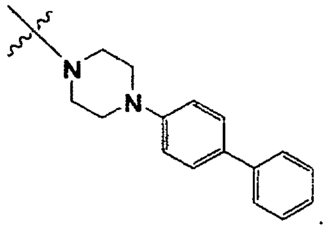

In yet another embodiment, A is

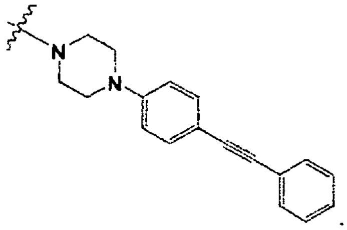

In another embodiment, A is

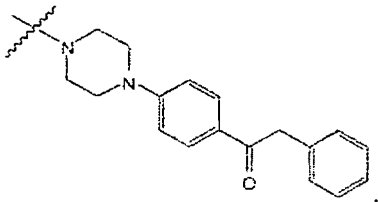

In yet another embodiment, A is Br or Cl.

In one embodiment, the compound of Formula (I) is:

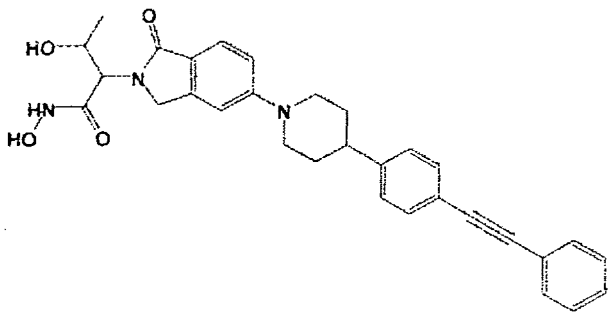

or a pharmaceutically acceptable salt, solvate or ester thereof.

In another embodiment, the compound of Formula (I) is:

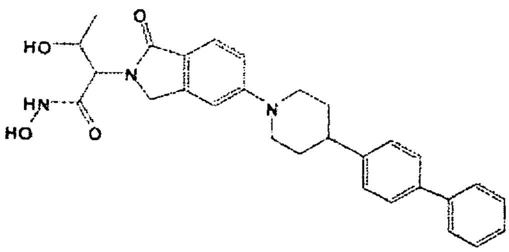

or a pharmaceutically acceptable salt, solvate or ester thereof.

In one embodiment, the compound of Formula (I) is:

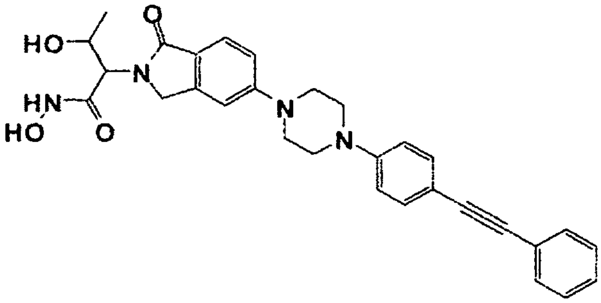

or a pharmaceutically acceptable salt, solvate or ester thereof.

In another embodiment, the compound of Formula (I) is:

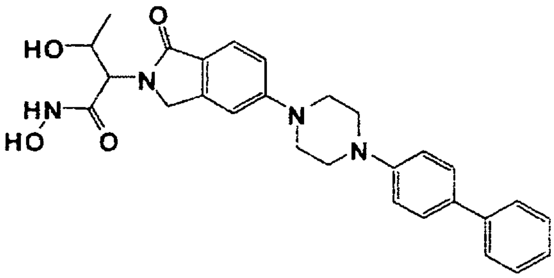

or a pharmaceutically acceptable salt, solvate or ester thereof.

In still another embodiment, the compound of Formula (I) is:

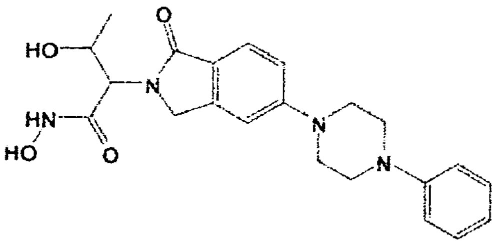

or a pharmaceutically acceptable salt, solvate or ester thereof.

in yet another embodiment, the compound of Formula (I) is:

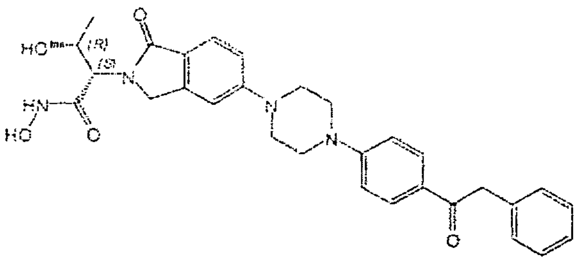

or a pharmaceutically acceptable salt, solvate or ester thereof.

In one embodiment, the compound of Formula (I) is:

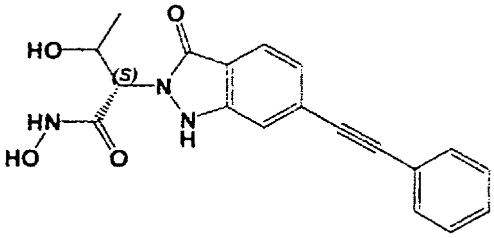

or a pharmaceutically acceptable salt, solvate or ester thereof.

In another embodiment, the compound of Formula (I) is:

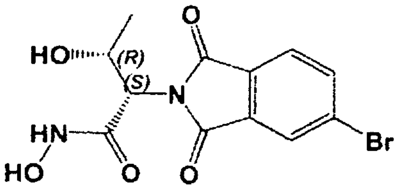

or a pharmaceutically acceptable salt, solvate or ester thereof.

In yet another embodiment, the compound of Formula (I) is:

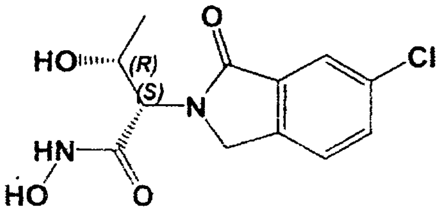

or a pharmaceutically acceptable salt, solvate or ester thereof.

Non-limiting examples of compounds of Formula I include:

| | |
|---|---|
| 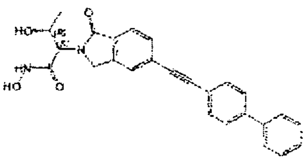 | 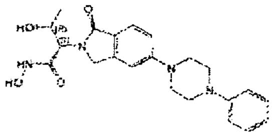 |

| | |
|---|---|
| 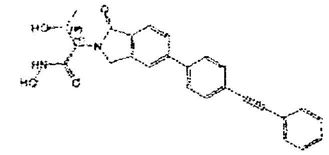 | 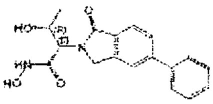 |
| 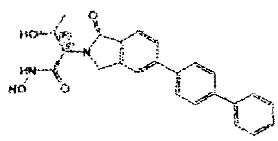 | 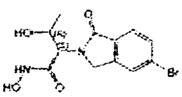 |
| 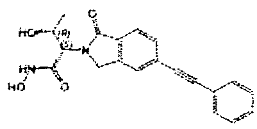 | 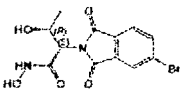 |
| 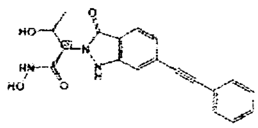 | 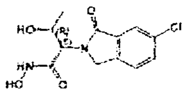 |
| 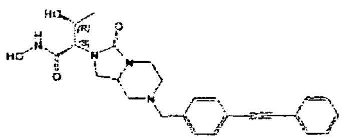 | 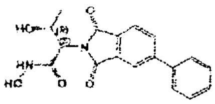 |
| 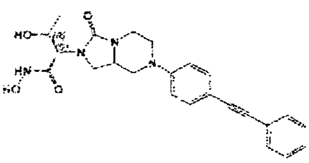 | 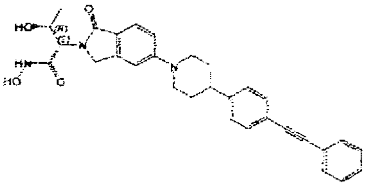 |
| 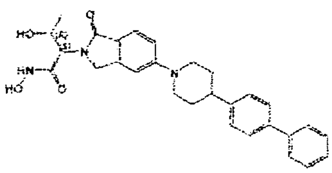 | 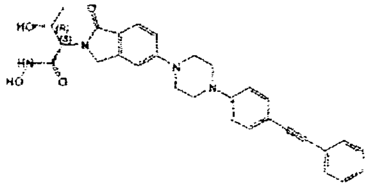 |

| | |
|---|---|
| 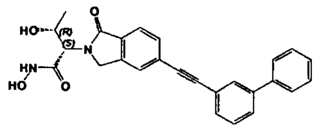 | 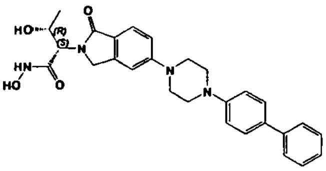 |
| 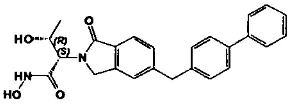 | 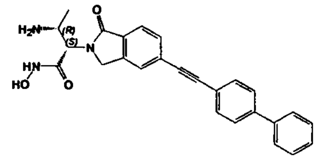 |
| 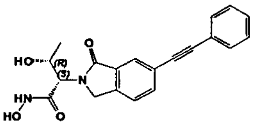 | 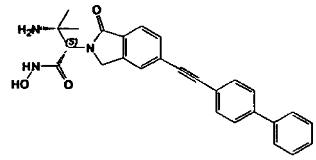 |
| 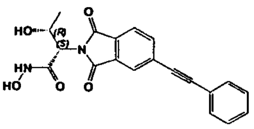 and | 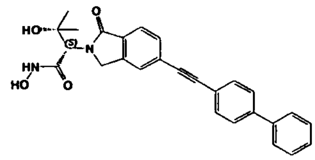 |

or a pharmaceutically acceptable salt, solvate or ester thereof.

As used above, and throughout this disclosure, the following terms, unless otherwise indicated, shall be understood to have the following meanings:

"Patient//subject" includes both human and animals.

"Mammal" means humans and other mammalian animals.

"Alkynyl" means an aliphatic hydrocarbon group containing at least one carbon-carbon triple bond and which may be straight or branched and comprising about 2 to about 15 carbon atoms in the chain. Preferred alkynyl groups have about 2 to about 12 carbon atoms in the chain; and more preferably about 2 to about 4 carbon atoms in the chain. Branched means that one or more lower alkyl groups such as methyl, ethyl or propyl, are attached to a linear alkynyl chain. "Lower alkynyl" means about 2 to about 6 carbon atoms in the chain which may be straight or branched. Examples of suitable alkynyl groups include ethynyl, propynyl, 2-butynyl and 3-methylbutynyl.

The prefix aza, oxa or thia before a heteroaryl root name means that at least a nitrogen, oxygen or sulfur atom respectively, is present as a ring atom. A nitrogen atom of a heteroaryl can be optionally oxidized to the corresponding N-oxide. Examples of suitable heteroaryls include pyridyl, pyrazinyl, pyrimidinyl, pyridone (including N-substituted pyridones), and pyridazinyl. The term "heteroaryl" also refers to partially saturated heteroaryl moieties. monocyclic cycloalkyls include cyclopropyl, cyclopentyl, cyclohexyl, cycloheptyl and the like. Non-limiting examples of suitable multicyclic cycloalkyls include 1-decalinyl, norbornyl, adamantyl and the like, as well as partially saturated species such as, for example, indanyl, tetrahydronaphthyl and the like.

"Cycloalkenyl" means a non-aromatic mono or multicyclic ring system comprising about 3 to about 10 carbon atoms, preferably about 5 to about 10 carbon atoms which contains at least one carbon-carbon double bond. Preferred cycloalkenyl rings contain about 5 to about 7 ring atoms. The cycloalkenyl can be optionally substituted with one or more "ring system substituents" which may be the same or different, and are as defined above. Non-limiting examples of suitable monocyclic cycloalkenyls include cyclopentenyl, cyclohexenyl, cyclohepta-1,3-dienyl, and the like. Non-limiting example of a suitable multicyclic cycloalkenyl is norbornylenyl.

"Haloalkyl" means an alkyl as defined above wherein one or more hydrogen atoms on the alkyl is replaced by

a halo group defined above. Non-limiting examples include trifluoromethyl, 2,2,2-trifluoroethyl, 2-chloropropyl and alike.

"Haloalkoxy" means an alkoxy group as defined below wherein one or more hydrogen atoms on the alkoxy is replaced by a halo/halogen group defined above. Non-limiting examples include trifluoromethoxy ($CF_3O$-), difluoromethoxy ($CHF_2O$-), 2,2,2-trifluoroethoxy ($CF_3CH_2O$-), 2-chloropropoxy ($CH_3CH(Cl)CH_2O$-) and alike.

"Halogen" or "halo" means fluorine, chlorine, bromine, or iodine. Preferred are fluorine, chlorine and bromine.

"Ring system substituent" means a substituent attached to an aromatic or non-aromatic ring system which, for example, replaces an available hydrogen on the ring system. Ring system substituents may be the same or different, each being independently selected from the group consisting of alkyl, aikenyl, alkynyl, aryl, heteroaryl, aralkyl, alkylaryl, heteroaralkyl, heteroarylalkenyl, heteroarylalkynyl, alkylheteroaryl, hydroxy, hydroxyalkyl, alkoxy, aryloxy, aralkoxy, acyl, aroyl, halo, nitro, cyano, carboxy, alkoxycarbonyl, aryloxycarbonyl, aralkoxycarbonyl, alkylsulfonyl, arylsulfonyl, heteroarylsulfonyl, alkylthio, arylthio, heteroarylthio, aralkyithio, heteroaralkylthio, cycloalkyl, heterocyclyl, -C(=N-CN)-$NH_2$, -C(=NH)-$NH_2$, - C(=NH)-NH(alkyl), $Y_1Y_2N$-, $Y_1Y_2N$-alkyl-, $Y_1Y_2NC(O)$-, $Y_1Y_2NSO_2$- and $SO_2NY_1Y_2$, wherein $Y_1$ and $Y_2$ can be the same or different and are independently

"Heterocyclyl" means a non-aromatic saturated monocyclic or multicyclic ring system in which one or more of the atoms in the ring system is an element other than carbon, for example nitrogen, oxygen or sulfur, alone or in combination. There are no adjacent oxygen and/or sulfur atoms present in the ring system. The prefix aza, oxa or thia before the heterocyclyl root name means that at least a nitrogen, oxygen or sulfur atom respectively is present as a ring atom. Any -NH in a heterocyclyl ring may exist protected such as, for example, as an -N(Boc), -N(CBz), -N(Tos) group and the like; such protections are also considered part of this invention. The nitrogen or sulfur atom of the heterocyclyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, and 1,4-dioxanyl.

"Heterocyclenyl" means a partially unsaturated monocyclic or partially unsaturated multicyclic ring system, in which one or more of the ring atoms is an element other than carbon, for example nitrogen, oxygen or sulfur, alone or in combination. Preferred heterocyclenyls also contain at least one -C=N as part of the ring. The prefix aza, oxa or thia before the heterocyclenyl root name means that at least a nitrogen, oxygen or sulfur atom respectively, is present as a ring atom. The nitrogen or sulfur atom of the heteroaryl can be optionally oxidized to the corresponding N-oxide, S-oxide or S,S-dioxide.

It should be noted that in hetero-atom containing ring systems of this invention, there are no hydroxyl groups on carbon atoms adjacent to a N, O or S, as well as there are no N or S groups on carbon adjacent to another heteroatom.

It should also be noted that tautomeric forms such as, for example, the moieties:

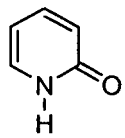
and
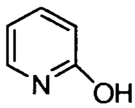

are considered equivalent in certain embodiments of this invention.

The term "substituted" means that one or more hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds. By "stable compound' or "stable structure" is meant a compound that is sufficiently robust to survive isolation to a useful degree of purity from a reaction mixture, and formulation into an efficacious therapeutic agent.

The term "optionally substituted" means optional substitution with the specified groups, radicals or moieties.

The term "isolated" or "in isolated form" for a compound refers to the physical state of said compound after being isolated from a synthetic process or natural source or combination thereof. The term "purified" or "in purified form" for a compound refers to the physical state of said compound after being obtained from a purification process or processes described herein or well known to the skilled artisan, in sufficient purity to be characterizable by standard analytical techniques described herein or well known to the skilled artisan.

It should also be noted that any carbon as well as heteroatom with unsatisfied valences in the text, schemes, examples and Tables herein is assumed to have the sufficient number of hydrogen atom(s) to satisfy the valences.

When a functional group in a compound is termed "protected", this means that the group is in modified form to preclude undesired side reactions at the protected site when the compound is subjected to a reaction. Suitable protecting groups will be recognized by those with ordinary skill in the art as well as by reference to standard textbooks such as, for example, T. W. Greene et al, Protective Groups in organic Synthesis (1991), Wiley, New York.

As used herein, the term "composition" is intended to encompass a product comprising the specified ingredients in the specified amounts, as well as any product which results, directly or indirectly, from combination of the specified

ingredients in the specified amounts.

"Solvate" means a physical association of a compound of this invention with one or more solvent molecules. This physical association involves varying degrees of ionic and covalent bonding, including hydrogen bonding. In certain instances the solvate will be capable of isolation, for example when one or more solvent molecules are incorporated in the crystal lattice of the crystalline solid. "Solvate" encompasses both solution-phase and isolatable solvates. Examples of suitable solvates include ethanolates, methanolates, and the like. "Hydrate" is a solvate wherein the solvent molecule is $H_2O$.

"Effective amount" or "therapeutically effective amount" as used herein, refers to an amount of Compound of Formula (I) and/or an additional therapeutic agent, or a composition thereof that is effective in producing the desired therapeutic, ameliorative, inhibitory or preventative effect when administered to a patient suffering from a Condition. In the combination therapies of the present invention, an effective amount can refer to each individual agent or to the combination as a whole, wherein the amounts of all agents administered are together effective, but wherein the component agent of the combination may not be present individually in an effective amount.

The compounds of Formula I can form salts which are also within the scope of this invention. Reference to a compound of Formula I herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula I contains both a basic moiety, such as a pyridine or imidazole, and an acidic moiety, such as a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful. Salts of the compounds of the Formula I may be formed, for example, by reacting a compound of Formula I with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, toluenesulfonates (also known as tosylates,) and the like. Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, lysine and the like. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Pharmaceutically acceptable esters of the present compounds include the following groups: (1) carboxylic acid esters obtained by esterification of the hydroxy groups, in which the non-carbonyl moiety of the carboxylic acid portion of the ester grouping is selected from straight or branched chain alkyl (for example, acetyl, n-propyl, t-butyl, or n-butyl), alkoxyalkyl (for example, methoxymethyl), aralkyl (for example, benzyl), aryloxyalkyl (for example, phenoxymethyl), aryl (for example, phenyl optionally substituted with, for example, halogen, $C_{1-4}$alkyl, or $C_{1-4}$alkoxy or amino); (2) sulfonate esters, such as alkyl- or aralkylsulfonyl (for example, methanesulfonyl); (3) amino acid esters (for example, L-valyl or L-isoleucyl); (4) phosphonate esters and (5) mono-, di- or triphosphate esters. The phosphate esters may be further esterified by, for example, a $C_{1-20}$ alcohol or reactive derivative thereof, or by a 2,3-di ($C_{6-24}$)acyl glycerol.

Compounds of Formula I, and salts and solvates thereof, may exist in their tautomeric form (for example, as an amide or imino ether). All such tautomeric forms are contemplated herein as part of the present invention.

All stereoisomers (for example, geometric isomers, optical isomers and the like) of the present compounds (including those of the salts, solvates and prodrugs of the compounds as well as the salts and solvates of the prodrugs), such as those which may exist due to asymmetric carbons on various substituents, including enantiomeric forms (which may exist even in the absence of asymmetric carbons), rotameric forms, atropisomers, and diastereomeric forms, are contemplated within the scope of this invention, as are positional isomers (such as, for example, 4-pyridyl and 3-pyridyl). Individual stereoisomers of the compounds of the invention may, for example, be substantially free of other isomers, or

may be admixed, for example, as racemates or with all other, or other selected, stereoisomers. The chiral centers of the present invention can have the S or R configuration as defined by the *IUPAC* 1974 Recommendations. The use of the terms "salt" and "solvate" and the like, is intended to equally apply to the salt and solvate of enantiomers, stereoisomers, rotamers, tautomers, positional isomers or racemates of the inventive compounds.

Polymorphic forms of the compounds of Formula I, and of the salts and solvates of the compounds of Formula I, are intended to be included in the present invention.

The compounds according to the invention have pharmacological properties; in particular, the compounds of Formula I are inhibitors of LpxC.

In one aspect, the invention provides a pharmaceutical composition comprising as an active ingredient at least one compound of Formula (I).

In another aspect, the invention provides a pharmaceutical composition of Formula (I) additionally comprising at least one pharmaceutically acceptable carrier.

In another aspect, the invention provides a method of treating disorders associated with LpxC, said method comprising administering to a patient in need of such treatment a pharmaceutical composition, which comprises a therapeutically effective amount of at least one compound of Formula (I).

In another aspect, the invention provides a use of a compound of Formula (I) for the manufacture of a medicament to treat disorders associated with LpxC.

The compounds of Formula I have antibacterial activitity and can be useful in the treatment of a microbial infection, including gram negative and gram positive infections.

In another aspect, the invention provides a method of preparing a pharmaceutical composition for treating the disorders associated with LpxC, said method comprising bringing into intimate contact at least one compound of Formula I and at least one pharmaceutically acceptable carrier.

In another aspect, the invention provides a pharmaceutical composition for treating disorders associated with LpxC, in a subject comprising, administering to the subject in need of such treatment a therapeutically effective amount of a compound of Formula I or a pharmaceutically acceptable salt, solvate, ester or isomer thereof.

In another aspect, the invention provides a compound of Formula I in purified form.

Also described is a method of treating a condition or disease mediated by LpxC (such as a microbial infection), in a subject comprising: administering to the subject in need of such treatment a therapeutically effective amount of at least one compound of Formula I or a pharmaceutically acceptable salt, solvate or isomer thereof.

Further described is a method for the treatment of a microbial infection in a mammal, comprising administering to said mammal a therapeutically effective amount of a compound of Formula I or a pharmaceutically acceptable salt, solvate or ester thereof.

In one embodiment, the microbe causing the infection is a bacteria, in another embodiment it is a fungus. In one embodiment, the microbial infection is a gram negative infection; in another embodiment, it is a gram positive infection.

Also described is a method for the treatment of a microbial infection in a mammal, comprising administering to said mammal a therapeutically effective amount of a compound of Formula I in combination with one or more additional antibacterial or antifungal agents. In one embodiment, said additional antibacterial agent is active against gram negative bacteria. In another embodiment, said additional antibacterial agent is active against gram positive bacteria.

In one embodiment, the compounds of Formula (I) can be administered to a subject to treat gram negative bacterial infections. They may also be given along with other antibiotics, such as the macrolides, e.g., erythromycin, rifampicin and azithromycin, to achieve or enhance the gram negative antibacterial activity, or with other non-macrolide antibiotics to achieve or enhance the spectrum or potency of the particular antibacterial agent against gram negative organisms.

Likewise, the compounds of Formula I can be used with other agents, which are in and of themselves useful in conjunction with antibacterial agents. For example, bacterial cell wall permeabilizing agents can be included. Representative examples of such compounds include EDTA, polymixin B nonapeptide, poly-L-lysine and neomycin. Other permeability enhancing agents known to those skilled in the art can be included herein as well.

In another embodiment, the bacterial infection treatable by the compounds of the present invention is caused by at least one organism selected from the group consisting of *Acinetobacter baumannii, Acinetobacter calcoaceticus, Acinetobacter haemolyticus, Acinetobacter hydrophila, Actinobacillus actinomycetemcomitans, Aeromonas hydrophila, Alcaligenes xylosoxidans, Bacteroides distasonis, Bacteroides fragilis, Bacteroides melaninogenicus, Bacteroides ovatus, Bacteroides thetaiotaomicron, Bacteroides vulgatus, Bartonella henselae, Bordetella pertussis, Branhamella catarrhalis, Brucella melitensis, Brucella abortus, Brucella canis, Burkholderia cepacia, Burkholderia mallei,Burkholderia pseudomallei, Campylobacter coli, Campylobacter fetus,*

*Campylobacter jejuni, Citrobacter diversus, Citrobacter freundii, Citrobacter koseri, Coxiella burnetli, Edwarsiella tarda, Ehrlichia chafeenis, Eikenella corrondens, Enterobacter aerogenes, Enterobacter agglomerans, Enterobacter cloacae, Escherichia coli, Flavobacterium meningosepticum, Francisella tularensis, Fusobacterium spp., Haemophilus ducreyi, Haemophilus influenzae,Haemophilus parainfluenzae, Helicobacter pylori, Kingella kingae, Klebsiella oxytoca,Klebsiella*

*ozaenae, Klebsiella pneumoniae, Klebsiella rhinoscleromatis, Legionella pneumophila, Moraxella catarrhalis, Morganella morganii, Neisseria gonorrhoeae, Neisseria meningitides, Pasteurella multocida, Plesiomonas shigelloides, Porphyromonas asaccharolytica, Porphyromonas gingivalis, Prevotella bivia,Prevotella buccae, Prevotella corporis, Prevotella endodontalis, Prevotella intermedia, Prevotella melaninogenica,Prevotella oralis, Proteus mirabilis, Proteus myxofaciens, Proteus penner, Proteus vulgaris, Providencia alcalifaciens, Providencia rettgeri, Providencia stuarfii, Pseudomonas aeruginosa, Pseudomonas fluorescens, Ricketsia prowozekii, Salmonella enterica, Serratia marcescens, Shigella boydii, Shigella dysenteriae, Shigella flexneri, Shigella sonnei, Stenotrophomonas maltophilia, Streptobacillus moniliformis, Vibrio alginolyticus, Vibrio cholerae, Vibrio parahaemolyticus, Vibrio vuluificus, Yersinia enterocolitica, Yersinia pestis,* and *Yersinia pseudotuberculosis.*

In another embodiment, the bacterial infection is caused by at least one organism selected from the group consisting of *Acinetobacter baumannii, Acinetobacter spp., Aeromonas hydrophila, Bacteroides fragilis, Bacteroides spp., Bordetella pertussis, Campylobacter jejuni, Campylobacter spp., Citrobacter freundii, Citrobacter spp., Enterobacter cloacae, Enterobacter spp., Escherichia coli, Fusobacterium spp., Haemophilus influenzae, Haemophilus parainfluenzae, Helicobacter pylori, Klebsiella pneumoniae, Klebsiella spp., Legionella pneumophila, Moraxella catarrhalis, Morganella morganii, Neisseria gonorrhoeae, Neisseria meningitides, Pasteurella multocida, Prevotella spp., Proteus mirabilis, Proteus spp., Providencia stuartii, Pseudomonas aeruginosa, Pseudomonas spp., Salmonella enterica, Salmonella typhi, Serratia marcescens, Shigella spp., Stenotrophomonas maltophilia, Vibrio cholerae, Vibrio spp.,* and *Yersinia spp.*

The standard LpxC assay consists of 0.2 nM LpxC enzyme, 1.0 μM UDP-3-*O*-(*R*-3-hydroxymyristoyl)-*N*-acetylglucosamine, and test compound, in assay buffer and 2% DMSO. Assay buffer is comprised of 25 mM HEPES, pH 7.3, 150 mM NaCl, 2.0 mM DTT, and 0.01 % BSA. The enzyme reaction is carried out in a 96-well assay plate, in a final volume of 102 μL. Solutions of test compounds are prepared in 100% DMSO. Reaction additions, in order, are (1) 2.0 μL compound solution , (2) 80 μL of assay buffer, (3) 10 μL of 10 μM UDP-3-*O*-(*R*-3-hydroxymyristoyl)-*N*-acetylglucosamine (in assay buffer) and, (4) 10 μL of LpxC enzyme (20 nM in assay buffer) to initiate the reaction. In positive control reactions, addition (1) has 2.0 μL of 100% DMSO (without compound); these reactions are used as the total signal (TSB) value. Reactions are incubated at room temperature for 60 minutes when 10 μL of 1 N HCl is added to stop the reaction. The plate is shaken by hand for 10 seconds to ensure complete quenching. Assay plates are sealed with foil tape, and stored at -80 °C for 24 - 48 hr prior to analysis.

The concentrations of substrate and product in the reaction mixtures are determined with BioTrove's proprietary RapidFire™ high-throughput mass spectrometry (HTMS). Assay mixtures are partially purified with reverse phase chromatography, where they are washed with water containing 5 mM ammonium formate and eluted onto the mass spectrometer in 80% acetonitrile, 20% water, and 5 mM ammonium formate. The mass spectrometry peak areas of the substrate and product are measured to determine the concentration of these analytes. The assay signal is the percentage of substrate that is converted to product. Percent inhibition, %I, in test samples is determined from the following equation:

$$\%I = 100 * \frac{(TSB - SampleSignal)}{(TSB)}.$$

Using this method, the following *E. coli* $IC_{50}$ (nM) data were obtained for selected Compounds of Formula (I):

Compounds 11 -14, 20, 21, 26, 31, 32, 36, 48 - 53, 65, 74, 79, 80, 88 and 91 had an $IC_{50}$ value of less than about 10 μM.
Compounds 11 - 14, 20, 21, 26, 31, 32, 36, 50, 52, 53, 65. 74, 79, 80, 88 and 91 had an $IC_{50}$ value of less than about 5 μM.
Compounds 11, 13, 31, 32, 36 and 65 had an $IC_{50}$ value of less than about 0.5μM.
Compounds 13, 31, 32 and 36 had an $IC_{50}$ value of less than about 0.05 μM.

The pharmaceutical compositions containing the active ingredient may be in a form suitable for oral use, for example, as tablets, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl

monostearate or glyceryl distearate may be employed. They may also be coated by the technique described in the U.S. Pat. Nos. 4,256,108; 4,166,452; and 4,265,874 to form osmotic therapeutic tablets for controlled release.

Formulations for orai use may also be presented as hard gelatin capsules wherein the active ingredients is mixed with an inert solid diluent, for example, calcium carbonate, calcium phosphate or kaolin, or a soft gelatin capsules where in the active ingredient is mixed with water or an oil medium, for example peanut oil, liquid paraffin or olive oil.

Aqueous suspensions contain the active material in admixture with excipients suitable for the manufacture of aqueous suspensions. Such excipients are suspending agents, for example, sodium carboxymethylcellulose, methylcellulose, hydroxypropylmethyl-cellulose, sodium alginate, polyvinyl-pyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example, heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example, polyethylene sorbitan monooleate. The aqueous suspensions may also contain one or more preservatives, for example, ethyl or n-propyl, p-hydroxybenzoate, one or more coloring agents, one or more flavoring agents, and one or more sweetening agents, such as sucrose, saccharin or aspartame.

Oily suspensions may be formulated by suspending the active ingredient in a vegetable oil, for example, arachis oil, olive oil, sesame oil or coconut oil, or in mineral oil such as liquid paraffin. The oily suspensions may contain a thickening agent, for example, beeswax, hard paraffin or cetyl alcohol. Sweetening agents such as those set forth above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, e.g., sweetening, flavoring and coloring agents, may also be present.

The pharmaceutical compositions of the invention may also be in the form of an oil-in-water emulsion. The oily phase may be a vegetable oil, e.g., olive oil or arachis oil, or a mineral oil, e.g., liquid paraffin or mixtures of these. Suitable emulsifying agents may be naturally-occurring phosphatides, e.g., soy beans, lecithin, and esters or partial esters derived from fatty acids and hexitol anhydrides, for example, sorbitan monscleate, and condensation products of the said partial esters with ethylene oxide, e.g., polyoxyethylene sorbitan monooleate. The emulsions may also contain sweetening and flavoring agents.

Syrups and elixirs may be formulated with sweetening agents, for example, glycerol, propylene glycol, sorbitol or sucrose. Such formulations may also contain demulcent, preservative, flavoring and coloring agents.

The pharmaceutical compositions may be in the form of a sterile injectable aqueous or oleagenous suspension. This suspension may be formulated according to the known art using those suitable dispersing or wetting agents and suspending agents, which have been mentioned above. The sterile injectable preparation may also be a sterile injectable solution or suspension in a non-toxic parenterally-acceptable diluent or solvent, e.g., as a solution in 1,3-butane diol. Among the acceptable vehicles and solvents that may be employed are water, Ringer's solution and isotonic sodium chloride solution. In addition, sterile fixed oils are conventionally employed as a solvent or suspending medium. For this purpose any bland fixed oil may be employed including synthetic mono- or diglycerides. In addition, fatty acids such as oleic acid find use in the preparation of injectables.

Compounds of the invention may also be administered in the form of suppositories for rectal administration of the drug. The compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

For topical use, creams, ointments, jellies, solutions or suspensions, etc., containing the compound of the invention are employed. (For purposes of this application, topical application shall include mouthwashes and gargle.)

The compounds for the present invention can be administered in the intranasal form via topical use of suitable intranasal vehicles, or via transdermal routes, using those forms of transdermal skin patches well known to those of ordinary skill in the art. To be administered in the form of a transdermal delivery system, the dosage administration will, of course, be continuous rather than intermittent throughout the dosage regimen. Compounds of the present invention may also be delivered as a suppository employing bases such as cocoa butter, glycerinated gelatin, hydrogenated vegetable oils, mixtures of polyethylene glycols of various molecular weights and fatty acid esters of polyethylene glycol.

The dosage regimen utilizing the compounds of the present invention is selected in accordance with a variety of factors including type, species, weight, sex and medical condition of the patient; the severity of the condition to be treated; the route of administration; the renal and hepatic function of the patient; and the particular compound thereof employed. A physician or veterinarian of ordinary skill can readily determine and prescribe the effective amount of the drug required to prevent, counter, arrest or reverse the progress of the condition. Optimal precision in achieving concentration of drug within the range that yields efficacy without toxicity requires a regimen based on the kinetics of the drug's availability to target sites. This involves a consideration of the distribution, equilibrium, and elimination of a drug. Preferably, doses of the compound of Formula I useful in the method of the present invention range from 0.01 to 1000 mg per day. More preferably, dosages range from 0.1 to 1000 mg/day. Most preferably, dosages range from 0.1 to 500 mg/day. For oral administration, the compositions are preferably provided in the form of tablets containing 0.01 to 1000 milligrams of the active ingredient, particularly 0.01, 0.05, 0.1, 0.5, 1.0, 2.5, 5.0, 10.0, 15.0, 25.0, 50.0, 100 and 500 milligrams of the active ingredient for the symptomatic adjustment of the dosage to the patient to be treated. An effective amount of the drug is ordinarily supplied at a dosage level of from about 0.0002 mg/kg to about 50 mg/kg of body weight per day. The range is more particularly from about 0.001 mg/kg to 1 mg/kg of body weight per day.

Advantageously, the active agent of the present invention may be administered in a single daily dose, or the total daily dosage may be administered in dividend doses of two, three or four time daily.

The amount of active ingredient that may be combined with the carrier materials to produce single dosage form will vary depending upon the host treated and the particular mode of administration.

It will be understood, however, that the specific dose level for any particular patient will depend upon a variety of factors including the age, body weight, general health, sex, diet, time of administration, route or administration, rate of excretion, drug combination and the severity of the particular disease undergoing therapy.

The compounds of Formula (I) may be produced by processes known to those skilled in the art and as shown in the following reaction schemes and in the preparations and examples described below. Alternate mechanistic pathways and analogous structures may be apparent to those skilled in the art. All kinds of isomeric forms of the compounds are considered to be within the scope of this invention.

## EXAMPLES

The following abbreviations are used in the procedures and schemes:

| | |
|---|---|
| ACN | Acetonitrile |
| AcOH | Acetic acid |
| Anh. | Anhydrous |
| Aq | Aqueous |
| BOC | tert-Butoxycarbonyl |
| °C | degrees Celsius |
| DCM | Dichloromethane |
| DIEA | Diisopropylethylamine |
| DMF | Dimethylformamide |
| DMSO-$d_6$ | Hexadeuterodimethylsulfoxide |
| EtOAc | Ethyl acetate |
| HATU | O-(7-Azabenzotriazole-1-yl)-N, N,N'N'-tetramethyluronium hexafluorophosphate |
| HPLC | High pressure liquid chromatography |
| LC-MS | Liquid Chromatography Mass Spectrometry |
| M | Molar |
| MeCN | Acetonitrile |
| MeOH | Methanol |
| min | Minutes |
| mg | Milligrams |
| MHz | Megahertz |
| ml | Milliliter |
| MS | Mass Spectroscopy |
| m/z | mass per charge |
| RT | Room temperature |
| THF | Tetrahydrofuran |
| TLC | Thin layer chromatography |
| $t_R$ | Retention time |
| X-Phos | 5-Bromo-4-chloro-3-indolyl Phosphate |

NMR spectra were acquired on a Mercuryplus 400 MHz NMR Spectrometer (Varian), using $CDCl_3$ or DMSO-$d_6$ as solvents. LC-MS data was obtained using an Agilent 1100 Series LC/MSD (quadrupole, API-ES (Atmospheric Pressure Interface Electrospray)) with a capillary voltage set to 3500 V and running in positive mode. Reported analytical HPLC (LC/MS) retention times were obtained using a C18 (150 x 4.6 mm) reverse-phase column eluting with a 5 or 10 minute

gradient of 0.1 % trifluoroacetic acid in water to 95:5 acetonitrile:water at a flow rate of 3 mL/min.

Purification via reverse phase chromatography was accomplished using a C18 reverse phase column with a gradient of 0.1 % trifluoroacetic acid in water to 95:5 acetonitrile:water at a flow rate of 20 mL/min. Samples were collected using a UV (Gilson, 254 nm) or mass spectra (Agilent 1100 Series LC/MSD model SL) signal.

Normal phase silica gel chromatography on a Biotage instrument was accomplished using a Quad UV System (P/N 07052) utilizing KP-SIL 32-63 um columns, 60A with flash cartridges 12+M or 25+M.

**Example 1:**

**Example 1A:**

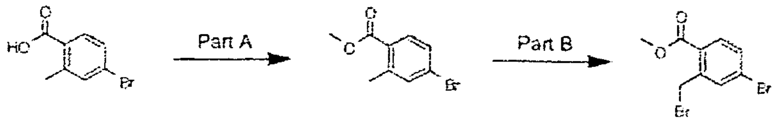


1 2 3

Part A:

To a solution of 4-bromo-2-methytber!zoic acid (1) (430 mg, 2 mmol) in MeCN (5 mL) and MeOH (5 mL) was added trimethylsilydiazomethane (2*M*, 3 mL, 6 mmol). The reaction mixture was stirred at room temperature for 20 minutes, quenched with the addition of AcOH (10 %) in MeOH (5 mL), and concentrated to afford crude compound 2. This was further purified by flash column chromatography, gradient elution (0 to 100 %) hexane / ethyl acetate, to afford compound 2 as a colorless oil (388 mg, 85 % yield).

Part B:

A solution of compound 2 (388 mg, 1.69 mmol), *N*-bromosuceinimide (NBS, 302 mg, 1.69 mmol) and benzoyl peroxide (12.3 mg, 0.05 mmol) in carbon tetrachloride (6 mL) was heated to reflux for 18 hours. LC-MS analysis indicated the reaction was complete. The reaction mixture was diluted with diethyl ether (10 mL) and passed through a plug of celite to remove precipitates. The filtrate was washed with saturated $NaHCO_3$, dried over magnesium sulfate, concentrated and purified by flash column chromatography, gradient elution (0 to 100 %) hexane / ethyl acetate, to afford compound 3 as a colorless oil (310 mg, 60 % yield). HPLC-MS $t_R$ = 2.00 min ($UV_{254\ nm}$); mass calculated for formula $C_9H_8Br_2O_2$ 305.9, observed LCMS m/z 306.9 (M+H).

**Example 1B:**

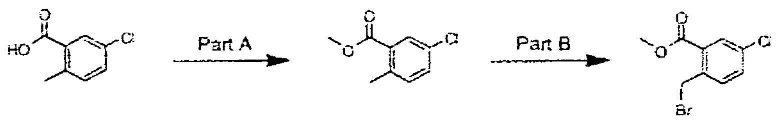


4 5 6

Compound 6 was prepared from 5-chloro-2-methylbenzoic acid (4) using the conditions described in Example 1A, Part A and Part B.

**Example 2:**

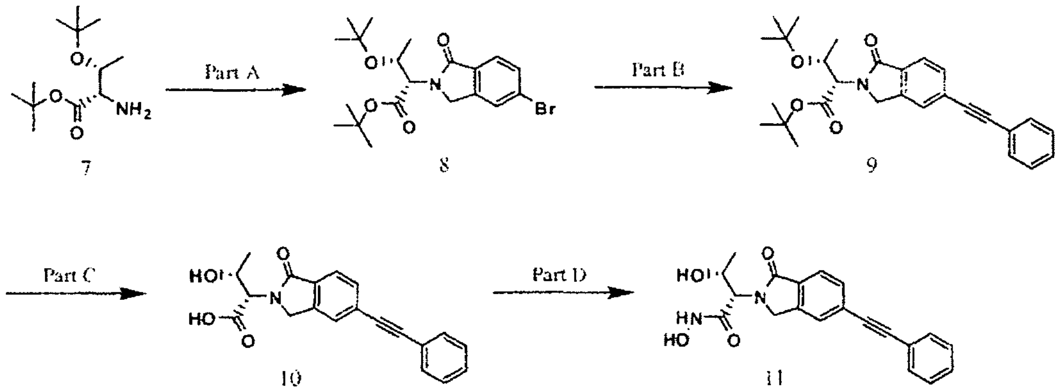


7 8 9 10 11

Part A:

A solution of O-*tert*-butyl-L-threonine *tert*-butyl ester hydrochloride (7) (433 mg, 1.62 mmol), compound 3 (550 mg, 1.8 mmol) and DIEA (1.9 mL, 9.72 mmol) in DMF (10 mL) was heated at 80 °C for 18 hours. LC-MS analysis indicated the reaction was complete. The volatiles were removed *in vacuo,* the residue re-dissolved in EtOAc and washed with 1*N* HCl. Drying over magnesium sulfate, concentration and purification by flash column chromatography, gradient elution (0 to 100 %) hexane /ethyl acetate, afforded compound 8 as a white solid (550 mg, 80 % yield). HPLC-MS $t_R$ = 2.55 min ($UV_{254\ nm}$); mass calculated for formula $C_{20}H2_{8}BrNO_{4}$ 425.1, observed LCMS m/z 426.1 (M+H).

Part B:

A solution of compound 8 (143 mg, 0.34 mmol) in acetonitrile (2 mL) was transferred to a Schlenk tube containing dichlorobis(acetonitrile)palladium (II) (0.87 mg, 3.4 μmol), X-Phos (5 mg, 10.2 μmol) and cesium carbonate (285 mg, 0.87 mmol) and the reaction mixture was stirred at room temperature under an inert atmosphere for 25 minutes. 100 μL of a solution containing phenylacetylene (69 mg, 0.67 mmol) in acetonitrile (1 mL) was added and the reaction mixture heated at 90 °C for 15 minutes. The phenylacetylene solution (100 μL) was added every 15 minutes and the reaction mixture was heated at 90° C for a total of 2.5 hours. LC-MS analysis indicated the reaction was complete. Water (3 mL) was added and the crude product extracted into ethyl acetate (5 mL). Drying over magnesium sulfate, concentration and purification by flash column chromatography, gradient elution (0 to 100 %) hexane / ethyl acetate, afforded compound 9 as a yellow solid (130 mg, 87 % yield). HPLC-MS $t_R$ = 2.60 min (($UV_{254\ nm}$); mass calculated for formula $C_{28}H_{33}NO_{4}$ 447.2, observed LCMS m/z 448.2 (M+H).

Part C:

Trifluoroacetic acid (5 mL) was added to compound 9 (50 mg, 0.11 mmol) and the resulting mixture stirred at room temperature for 1 hour. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 10 as a brown solid. HPLC-MS $t_R$ = 1.65 min ($UV_{254\ nm}$); mass calculated for formula $C_{20}H_{17}NO_{4}$ 335.1, observed LCMS m/z 336.1 (M+H).

Part D:

To a solution of compound 10 (37 mg, 0.11 mmol) and HATU (50 mg, 0.13 mmol) in DMF (2 mL) was added DIEA (57 μL, 0.33 mmol) and *O*-(*tert*-butyldimethylsilyl) hydroxylamine (19 mg, 0.13 mmol). The reaction mixture was stirred at room temperature for 18 hours. LC-MS analysis indicated the reaction was complete. The volatiles were removed *in vacuo* and the resulting residue purified by Prep.HPLC to afford compound 11 (10.5 mg, 28 %) as an off white solid.

The compounds in table-1 (11-14) were synthesized using this procedure described in example-2.

**Table-1**

| Cmpd # | Structure | Exact mass | MS m/z ($M^{+}$+H) | Ret. Time (min) |
|---|---|---|---|---|
| 11 | 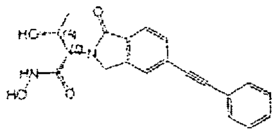 | 350.1 | 351.1 | 3.40 |
| 12 | 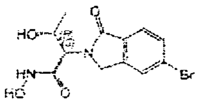 | 328.0 | 329.0 | 2.27 |
| 13 | 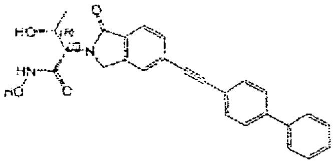 | 426.2 | 427.2 | 4.32 |
| 14 | 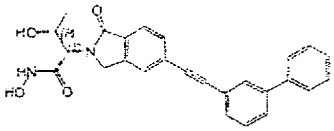 | 426.2 | 427.2 | 4.27 |

**Example 3:**

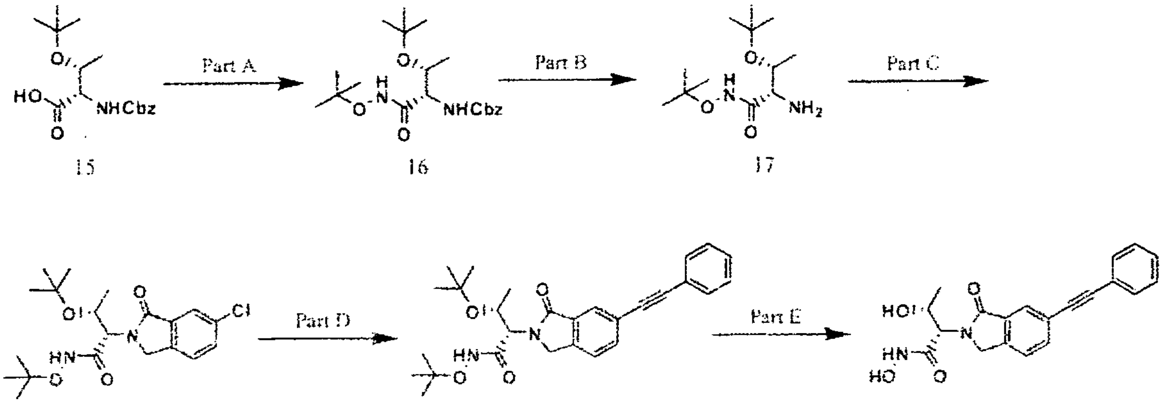


15 16 17 18 19 20

Part A:

Compound 16 (600 mg, 68 %) was prepared from the reaction of *N*-benzyloxycarbonyl-*O*-*tert*-butyl-L-threonine hydrochloride (720 mg, 2.32 mmol) and *tert*-butyl-O-hydroxylamine (351 mg, 2.78 mmol) using the peptide coupling conditions described in Example 2, Part D. HPLC-MS $t_R$ = 1.92 min ($UV_{254\ nm}$); mass calculated for formula $C_{20}H_{32}N_2O_5$ 380.2, observed LCMS m/z 381.2 (M+H).

Part B:

A solution of compound 16 (600 mg, 1.58 mmol) and palladium on charcoal (10 %) in EtOAc (20 mL) was subjected to hydrogenation for 18 hours. LC-MS analysis indicated the reaction was complete. The reaction mixture was filtered by passing through celite, and evaporated to afford crude compound 17 as a white solid (320 mg, 82 %). HPLC-MS $t_R$ = 1.00 min ($UV_{254\ nm}$); mass calculated for formula $C_{12}H_{26}$ $N_2O_3$ 246.2, observed LCMS m/z 247.3 (M+H).

Part C:

Compound 18 (120 mg, 66 %) was prepared from the reaction of compound 17 (113 mg, 0.46 mmol) and compound 6 (120 mg, 0.46 mmol) using the condensation conditions described in Example 2, Part A. HPLC-MS $t_R$ = 1.95 min ($UV_{254\ nm}$); mass calculated for formula $C_{20}H_{29}ClN_2O_4$ 396.2, observed LCMS m/z 397.2 (M+H).

Part D:

Compound 19 (80 mg, 57 %) was prepared from the reaction of compound 18 (120 mg, 0.30 mmol) and phenylacetylene (62 mg, 0.61 mmol) using the Sonagashira coupling conditions described in Example 2, Part B. HPLC-MS $t_R$ = 2.30 min ($UV_{254\ nm}$); mass calculated for formula $C_{28}H_{34}N_2O_4$ 462.3, observed LCMS m/z 463.3 (M+H).

Part E:

Trifluoroacetic acid (2 mL) was added to compound 19 (30 mg, 0.065 mmol) and the resulting mixture stirred at room temperature for 18 hours. LC-MS analysis indicated hydrolysis was complete. The volatiles were removed *in vacuo* and the resulting residue purified by Prep.HPLC to afford compound 20 (7.8 mg, 35 %) as an off white solid.
The compounds 20 and 21 were synthesized using the procedure described in example-3

**Table-2:**

| Cmpd # | Structure | Exact mass | MS m/z ($M^+$+H) | Ret. Time (min) |
|---|---|---|---|---|
| 20 | 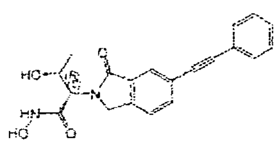 | 350.1 | 351.1 | 3.52 |
| 21 | 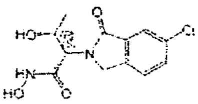 | 284.1 | 285.1 | 2.02 |

Example 4:

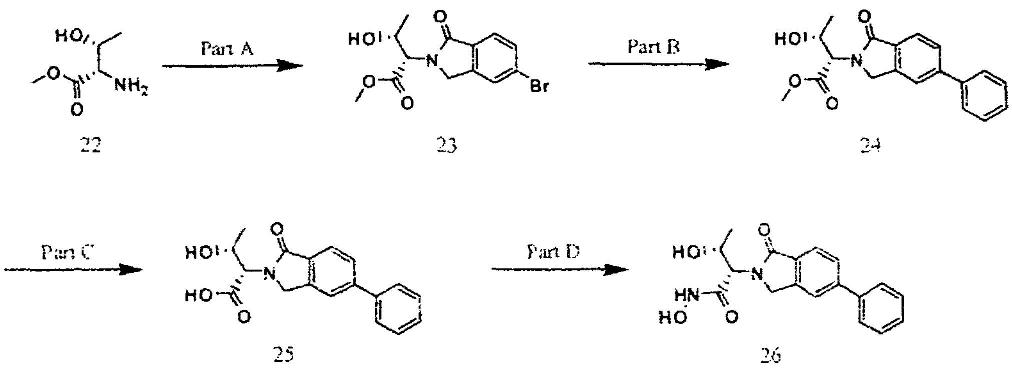

22 23 24

25 26

Part A:

Compound 23 (50 mg, 75 %) was prepared from the reaction of threonine methyl ester hydrochloride (35 mg, 0.2 mmol) and compound 6 (100 mg, 0.33 mmol) using the condensation conditions described in Example 2, Part A. HPLC-MS $t_R$ = 1.45 min ($UV_{254\ nm}$); mass calculated for formula $C_{13}H_{14}BrNO_4$ 327.0, observed LCMS m/z 328.0 (M+H).

Part B:

To a mixture of compound 23 (28 mg, 0.086 mmol), potassium phosphate (55 mg, 0.26 mmol) and dichloro[1,1'-bis(diphenylphosphino)ferrocene]palladium (II) dichloromethane adduct (6.3 mg, 8.6 μmol) in dioxane (1 mL) was added phenylboronic acid (12.5 mg, 0.1 mmol). The reaction vessel was flushed with argon, and the reaction mixture heated at 80 °C for 18 hours. LC-MS analysis of the reaction indicated that the reaction was complete. Ethyl acetate (3 mL) was added, and the precipitates removed by passing through a plug of celite. The filtrate was concentrated, and the crude residue purified by flash column chromatography, gradient elution (0 to 100 %) hexane / ethyl acetate, to afford compound 24 as a white solid (20 mg, 71 % yield). HPLC-MS $t_R$ = 1.61 min (($UV_{254\ nm}$); mass calculated for formula $C_{19}H_{19}NO_4$ 325.1, observed LCMS m/z 326.1 (M+H).

Part C:

A solution containing compound 24 (20 mg, 0.062 mmol) and lithium hydroxide (1M, 68 μL, 0.068 mmol) in THF (2 mL) and water (1 mL) was stirred at room temperature for 1 hour. LC-MS analysis indicated that the hydrolysis was complete. The reaction mixture was acidified to p*H* 4.0 with 1*N* HCl, and the crude product extracted into EtOAc (2x10 mL). Drying over magnesium sulfate and concentration afforded compound 25 as a white solid (18 mg, 94 %). HPLC-MS $t_R$ = 1.42 min ($UV_{254\ nm}$); mass calculated for formula $C_{18}H_{17}NO_4$ 311.1, observed LCMS m/z 312.1 (M+H).

Part D:

Compound 26 was prepared from compound 25 using the peptide coupling conditions described in Example 2, Part D.

The compound, 26, (Table-3) was synthesized using the procedure described in example 4

**Table- 3**

| Cmpd # | Structure | Exact mass | M m/z ($M^+$+H) | Ret. Time (min) |
|---|---|---|---|---|
| 26 | 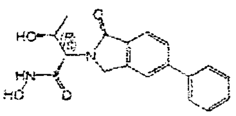 | 326.1 | 327.1 | 2.99 |

Example 5:

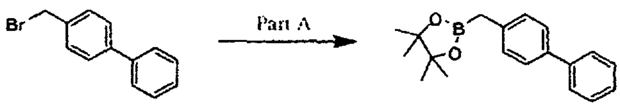


27 28

Part A:

A flask containing a mixture of 4-bromomethylbiphenyl (27) (150 mg, 0.61 mmol), potassium carbonate (252 mg, 1.82 mmol), bis(pinacolato)diboron (185 mg, 0.73 mmol) and tetrakis(triphenylphosphine)palladium (0) (35 mg, 0.03 mmol) in dioxane (3 mL) was flushed with argon, and the reaction mixture heated at 100 °C for 6 hours. LC-MS analysis of the reaction indicated that the reaction was complete. Ethyl acetate (5 mL) was added, and the precipitates removed by passing through a plug of celite. The filtrate was concentrated, and the crude residue purified by flash column chromatography, gradient elution (0 to 100 %) hexane / ethyl acetate, to afford compound 28 as a white solid (61 mg, 86 % yield). HPLC-MS $t_R$ = 2.40 min ($UV_{254\ nm}$); mass calculated for formula $C_{19}H_{23}BO_2$ 294.2, observed LCMS m/z 295.2 (M+H).

**Example 6:**

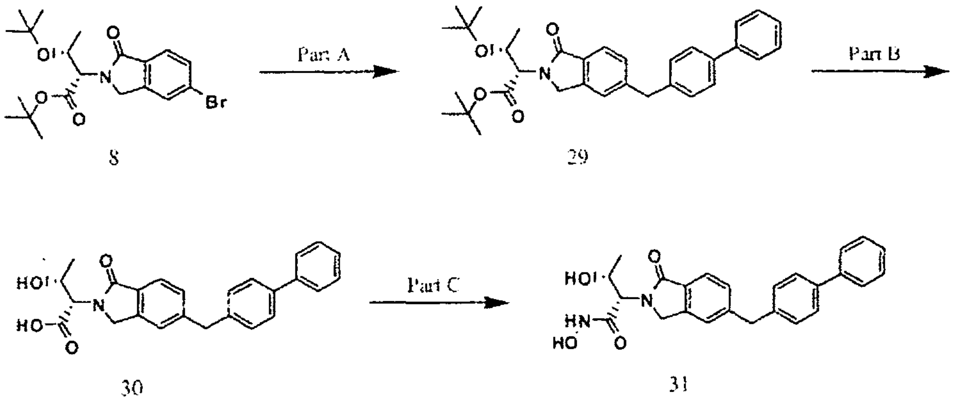


8 29 30 31

Part A:

Compound 29 (39 mg, 54 %) was prepared from the reaction of compound 8 (60 mg, 0.14 mmol) and compound 28 (83 mg, 0.28 mmol) using the Suzuki coupling conditions described in Example 4, Part B. HPLC-MS $t_R$ = 2.75 min (($UV_{254\ nm}$); mass calculated for formula $C_{33}H_{39}NO_4$ 513.3, observed LCMS m/z 514.3 (M+H).

Part B:

Compound 30 (29 mg, 95 %) was prepared from compound 29 (39 mg, 0.076 mmol) using the hydrolysis conditions described in Example 2, Part C. HPLC-MS $t_R$ = 2.00 min ($UV_{254\ nm}$); mass calculated for formula $C_{25}H_{23}NO_4$ 401.2, observed LCMS m/z 402.2 (M+H).

Part C:

Compound 31was prepared from compound 30 using the peptide coupling conditions described in Examples 2, Part D.

The compounds, 31 and 32 (Table-4) were synthesized using the procedure described example-6:

**Table- 4**

| Cmpd # | Structure | Exact mass | MS m/z ($M^{+}$+H) | Ret. Time (min) |
|---|---|---|---|---|
| 31 | 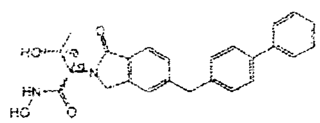 | 416.2 | 417.2 | 4.78 |
| 32 | 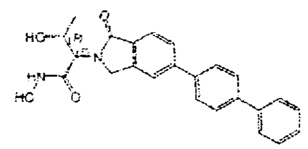 | 402.2 | 403.2 | 4.66 |

**Example 7:**

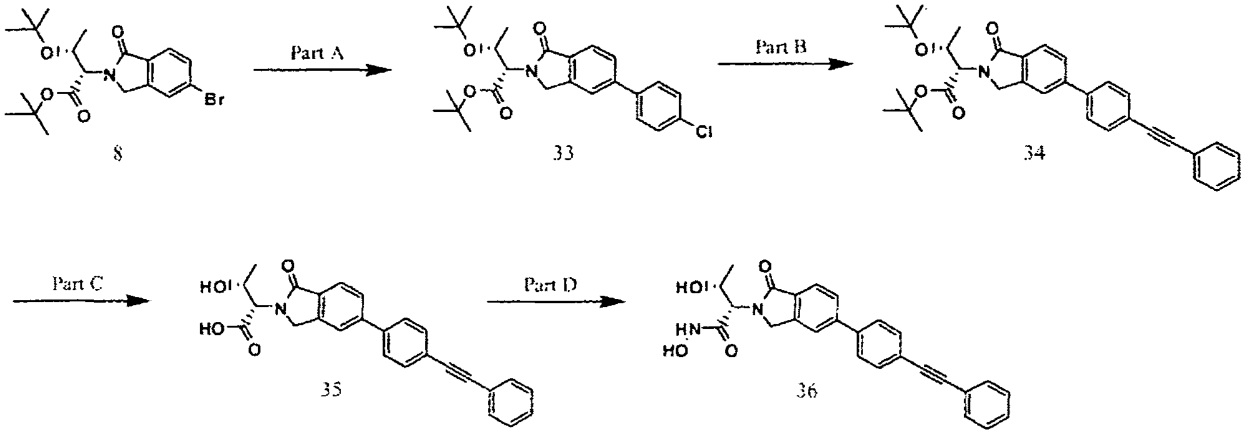

8
33
34
35
36

Part A:

Compound 33 (30 mg, 57 %) was prepared from the reaction of compound 8 (50 mg, 0.12 mmol) and 4-chlorophenylboronic acid (37 mg, 0.24 mmol) using the Suzuki coupling conditions described in Example 4, Part B. HPLC-MS $t_R$ = 2.86 min ($UV_{254\ nm}$); mass calculated for formula $C_{25}H_{32}ClNO_4$ 457.2, observed LCMS m/z 458.2 (M+H).

Part B:

Compound 34 (20 mg, 59 %) was prepared from the reaction of compound 33 (30 mg, 0.065 mmol) and phenylacetylene (13 mg, 0.13 mmol) using the Sonagashira coupling conditions described in Example 2, Part B. HPLC-MS $t_R$ = 2.66 min ($UV_{254\ nm}$); mass calculated for formula $C_{34}H_{37}N_0O_4$ 523.3, observed LCMS m/z 524.2 (M+H).

Part C:

Compound 35 (7.3 mg, 46 %) was prepared from compound 34 (20 mg, 0.038 mmol) using the hydrolysis conditions described in Example 2, Part C. HPLC-MS $t_R$ = 1.97 min ($UV_{254\ nm}$); mass calculated for formula $C_{26}H_{21}NO_4$ 411.1, observed LCMS m/z 412.1 (M+H).

Part D:

Compound 36 was prepared from compound 35 using the peptide coupling conditions described in Example 2, Part D.
The compound, 36, was synthesized using the procedure described in example 7

**Table- 5**

| Cmpd # | Structure | Exact mass | MS m/z ($M^+$+H) | Ret. Time (min) |
|---|---|---|---|---|
| 36 | 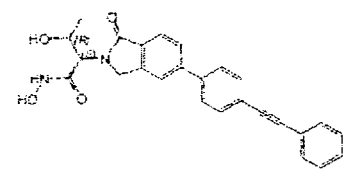 | 426.2 | 427.2 | 4.26 |

**Example 8:**

**Example 8A:**

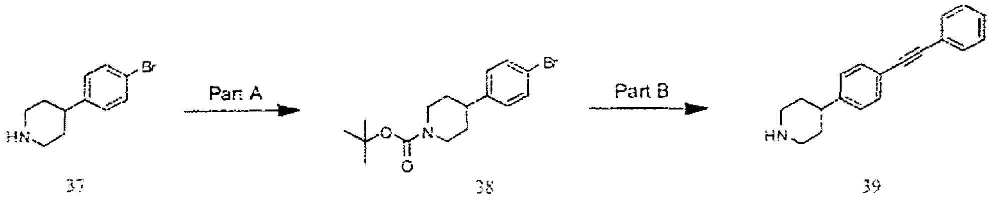


37 38 39

Part A:

A mixture of 4-(4-bromophenyl)piperidine (37) (960 mg, 4.0 mmol) and di-*tert*-butyl dicarbonate (960 mg, 4.4 mmol) at 0 °C in DCM (10 mL) was warmed to room temperature and stirred for 3 hours. LC-MS analysis indicated the reaction was complete. Dichloromethane (10 mL) was added and the solution washed with 1*N* HCl (10 mL). Drying over magnesium sulfate, concentration and purification by flash column chromatography, gradient elution (0 to 100 %) hexane / ethyl acetate, afforded compound 38 as a white solid (1.36 g, 100 % yield). HPLC-MS $t_R$ = 2.50 min ($UV_{254\ nm}$), mass calculated for formula $C_{16}H_{22}BrNO_2$ 339.1, observed LCMS m/z 284.1 (M+H-$^tBu$).

Part B:

A solution of compound 38 (600 mg, 1.76 mmol) in acetonitrile (5 mL) was transferred to a Schlenk tube containing dichlorobis(acetonitrile)palladium (II) (4.6 mg, 17.6 □mol), X-Phos (25 mg, 52.9 μmol) and cesium carbonate (1.5 g, 4.59 mmol) and the reaction mixture was stirred at room temperature under an inert atmosphere for 25 minutes. 100 μL of a solution containing phenylacetylene (360 mg, 3.52 mmol) in acetonitrile (2 mL) was added and the reaction mixture heated at 90°C for 15 minutes. The phenylacetylene solution (100 μL) was added every 15 minutes and the reaction mixture was heated at 90 °C for a total of 2.5 hours. LC-MS analysis indicated the reaction was complete. Water (6 mL) was added and the crude product extracted into ethyl acetate (10 mL). Drying over magnesium sulfate, concentration and purification by flash column chromatography, gradient elution (0 to 100 %) hexane / ethyl acetate, afforded BOC-protected compound 39 as a yellow solid (546 mg, 86 % yield). HPLC-MS $t_R$ = 2.70 min ($UV_{254\ nm}$); mass calculated for formula $C_{24}H_{27}NO_2$ 361.2, observed LCMS m/z 306.2 (M+H-$^tBu$).

The BOC-protecting group was hydrolyzed by the addition of trifluoroacetic acid (5 mL) and the resulting mixture stirred at room temperature for 1 minute. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 39. HPLC-MS $t_R$ = 1.22 min ($UV_{254\ nm}$); mass calculated for formula $C_{19}H_{19}N$ 261.2, observed LCMS m/z 262.2 (M+H).

**Example 8B:**

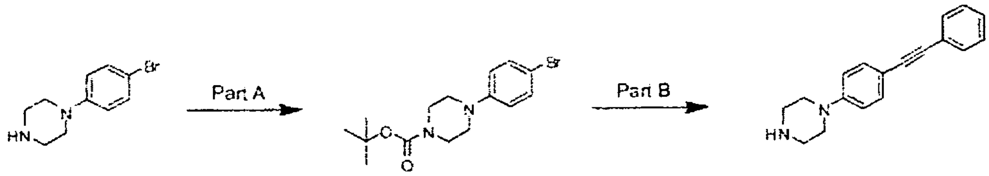


40 41 42

Compound 42 was prepared from 1-(4-bromophenyl)piperazine (40) using the conditions described in Example 8A, Part A and Part B. HPLC-MS $t_R$ = 1.19 min ($UV_{254\ nm}$); mass calculated for formula $C_{18}H_{18}N_2$ 262.2, observed LCMS m/z 263.1 (M+H).

**Example 8C:**

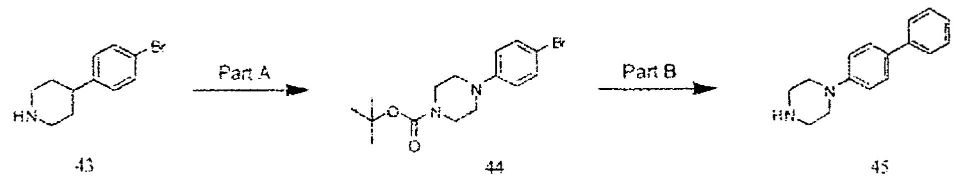


43 44 45

Part A:

Compound 44 was prepared from 4-(4-bromophenyl) piperidine (43) using the conditions described in Example 8A, Part A. HPLC-MS $t_R$ = 2.61 min (($UV_{254\ nm}$); mass calculated for formula $C_{16}H_{22}BrNO_2$ 339.1, observed LCMS m/z 284.0 (M+H-$^tBu$).

Part B:

Compound 45 was prepared from the reaction of compound 44 and phenylboronic acid using the Suzuki coupling conditions described in Example 4, Part B. The BOC-protecting group was hydrolyzed by the addition of trifluoroacetic acid (5 mL) and the resulting mixture stirred at room temperature for 1 minute. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 45. HPLC-MS $t_R$ = 1.24 min ($UV_{254\ nm}$); mass calculated for formula $C_{17}H_{19}N$ 237.2, observed LCMS m/z 238.3 (M+H).

**Example 9:**

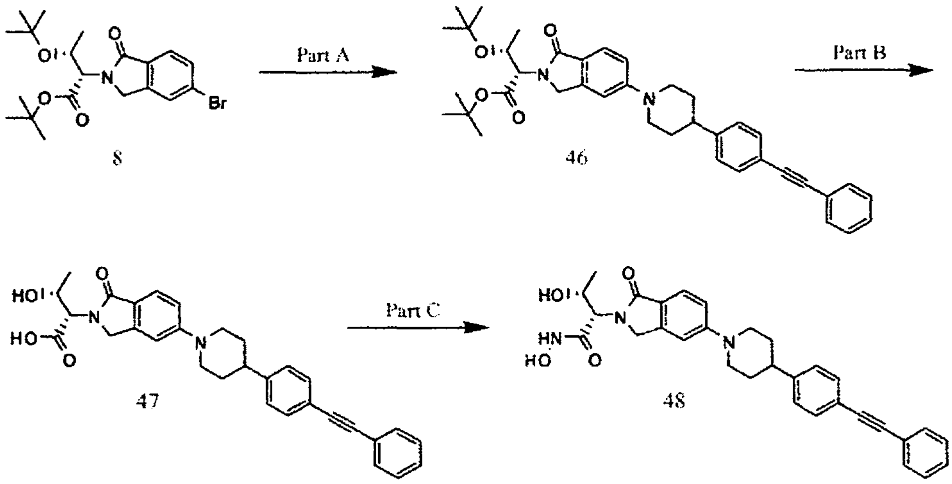


8 46 47 48

Part A:

A flask containing a mixture of compound 8 (80 mg, 0.19 mmol), compound 39 (74 mg, 0.28 mmol), potassium phosphate (120 mg, 0.56 mmol), X-Phos (9 mg, 18.8 μmol) and tris (dibenzylideneacetone) dipalladium (0) (8.6 mg, 9.4 μmol) in dioxane (3 mL) was flushed with argon, and the reaction mixture heated at 100 °C for 18 hours. LC-MS analysis of the reaction indicated that the reaction was complete. Ethyl acetate (5 mL) was added, and the precipitates removed by passing through a plug of celite. The filtrate was concentrated, and the crude residue purified by flash column chromatography, gradient elution (0 to 100 %) hexane / ethyl acetate, to afford compound 46 as a white solid (59 mg, 52 % yield). HPLC-MS $t_R$ = 2.88 min ($UV_{254}$ nm); mass calculated for formula $C_{39}H_{46}N_2O_4$ 606.3, observed LCMS m/z 607.3 (M+H).

Part B:

Compound 47 (42 mg, 88 %) was prepared from compound 46 (59 mg, 0.097 mmol) using the hydrolysis conditions described in Example 2, Part C. HPLC-MS $t_R$ = 2.14 min (($UV_{254\ nm}$); mass calculated for formula $C_{31}H_{30}N_2O_4$

494.2, observed LCMS m/z 495.3 (M+H).

Part D:

Compound 48 was prepared from compound 47 using the peptide coupling conditions described in Example 2, Part D.

The following compounds, 48 to 53 (Table-6) were synthesized using the procedure described in example-9

**Table-6**

| Cmpd # | Structure | Exact mass | MS m/z ($M^{+}$+ H) | Ret. Time (min) |
|---|---|---|---|---|
| 48 | 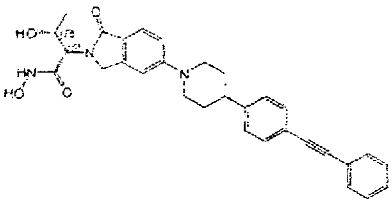 | 509.2 | 510.2 | 5.46 |
| 49 | 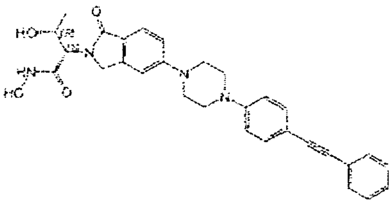 | 510.2 | 511.2 | 4.69 |
| 50 | 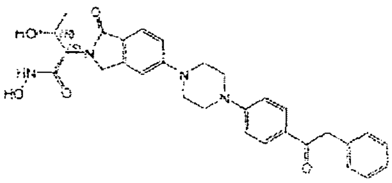 | 528.2 | 529.2 | 4.46 |
| 51 | 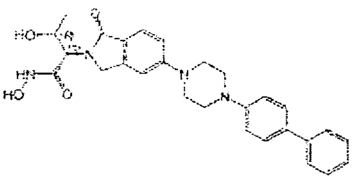 | 486.2 | 487.3 | 4.60 |
| 52 | 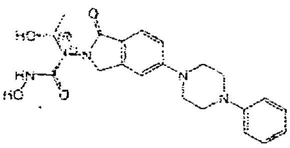 | 410.2 | 411.2 | 2.98 |
| 53 | 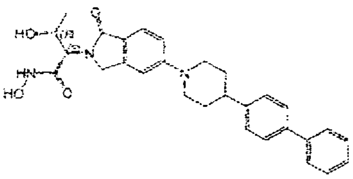 | 485.2 | 486.2 | 4.96 |

**Example 10:**

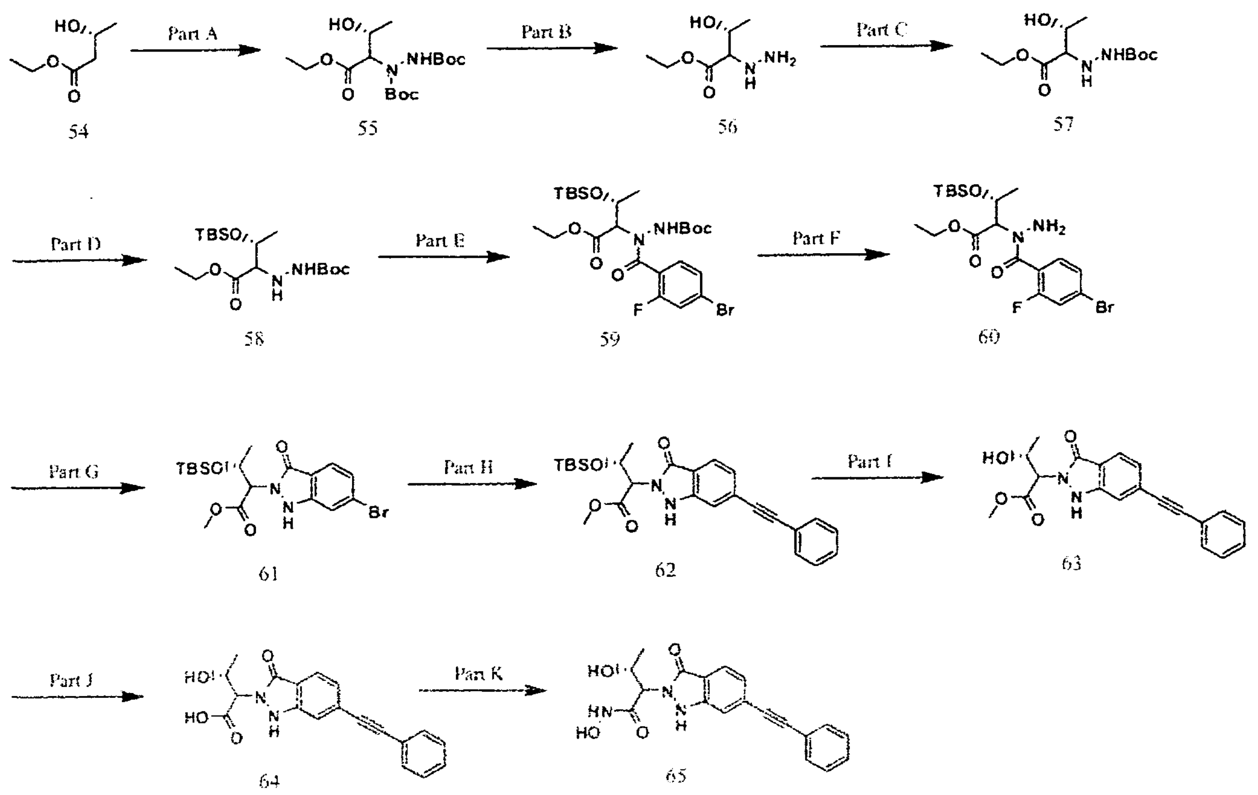


54 55 56 57 58 59 60 61 62 63 64 65

Part A:

A solution of lithium diisopropylamide (LDA, 1.8M, 14.3 mL, 25.7 mmol) in THF (30 mL) was cooled to -60 °C under an argon atmosphere. (*R*)-Ethyl 3-hydroxybutanoate (800 μL, 6.11 mmol) was diluted with THF (5 mL) and the resulting solution transferred to the stirring lithium diisopropylamide solution. The reaction mixture was warmed to - 20 °C over 30 minutes. The resulting dianion was cooled to -78 °C and a solution of di-*tert*-butylazodicarboxylate (3.52 g, 15.3 mmol) in THF (7 mL) slowly added. The reaction mixture was stirred at -78 °C for a further 10 minutes before quenching with AcOH (2.1 mL, 36.7 mmol). The reaction mixture was stirred at -78 °C for an additional 15 minutes, warmed to room temperature, diluted with water and extracted with EtOAc. Drying over magnesium sulfate, concentration and purification by flash column chromatography, gradient elution (0 to 100 %) hexane / ethyl acetate, afforded the separation of both diastereomers of compound 55 as a colorless oil (2.0 g, 91 % yield). Diastereomer 1: HPLC-MS $t_R$ = 1.94 min ($UV_{254\ nm}$); mass calculated for formula $C_{16}H_{30}N_2O_7$ 362.2, observed LCMS m/z 385.2 (M+Na). Diastereomer 2: HPLC-MS $t_R$ = 2.10 min ($UV_{254\ nm}$); mass calculated for formula $C_{16}H_{30}N_2O_7$ 362.2, observed LCMS m/z 385.2 (M+Na).

Part B:

Trifluoroacetic acid (2 mL) was added to a stirring solution of compound 55 (500 mg, 1.38 mmol) in DCM (2 mL) and the resulting mixture stirred at room temperature for 30 minutes. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 56. HPLC-MS $t_R$=0.17 min ($UV_{254\ nm}$); mass calculated for formula $C_6H_{14}N_2O_3$ 162.1, observed LCMS m/z 163.1 (M+H).

Part C:

Compound 57 was prepared from compound 46 using the conditions described in Example 8A, Part A. Two regioisomers were isolated. Regioisomer 1 (desired): HPLC-MS $t_R$ = 1.29 min ($UV_{254\ nm}$): mass calculated for formula $C_{11}H_{22}N_2O_5$ 262.2, observed LCMS m/z 285.2 (M+Na). Regioisomer 2: HPLC-MS $t_R$ = 1.38 min ($UV_{254\ nm}$); mass calculated for formula $C_{11}H_{22}N_2O_5$ 262.2, observed LCMS m/z 285.2 (M+Na).

Part D:

A solution of compound 57 (230 mg, 0.88 mmol), imidazole (174 mg, 2.64 mmol) and *tert*-butyldimethylsilyl chloride (265 mg, 1.76 mmol) in DMF (10 mL) was stirred at room temperature for 18 hours. LC-MS analysis indicated the reaction was complete. The reaction was quenched with water and extracted with EtOAc. Drying over magnesium sulfate and concentration afforded crude compound 58 as a colorless oil (314 mg, 95 %). HPLC-MS $t_R$ = 2.49 min ($UV_{254\ nm}$); mass calculated for formula $C_{17}H_{36}N_2O_5Si$ 376.2, observed LCMS m/z 321.3 (M+H-$^tBu$).

Part E:

4-bromo-2-fluorobenzoyl chloride (271 mg, 1.14 mmol) was added to a solution of compound 58 (314 mg, 0.84 mmol) and DIEA (436 μL, 2.5 mmol) in THF (5 mL) and the reaction mixture heated at 60 °C for 1 hour. LC-MS analysis of the reaction indicated that the reaction was complete. The reaction was quenched with the addition of 1 N HCl and extracted with EtOAc. Drying over magnesium sulfate and concentration afforded crude compound 59 which was further purified by flash column chromatography, gradient elution (0 to 100 %) hexane / ethyl acetate (320 mg, 66 %). HPLC-MS $t_R$ = 2.80 min ($UV_{254\ nm}$); mass calculated for formula $C_{24}H_{38}BrFN_2O_4Si$ 576.2, observed LCMS m/z 599.2 (M+Na).

Part F:

Trifluoroacetic acid (2 mL) was added to compound 59 (50 mg, 0.087 mmol) and the resulting mixture stirred at room temperature for 5 minutes. LC-MS analysis indicated BOC-hydrolysis was complete. The volatiles were removed *in vacuo* and the resulting residue re-dissolved in EtOAc (10 mL) and washed with saturated $NaHCO_3$. Drying over magnesium sulfate and concentration afforded crude compound 60 (40 mg, 97 %) as a white solid. HPLC-MS $t_R$ = 2.57 min ($UV_{254\ nm}$); mass calculated for formula $C_{19}H_{30}BrFN_2O_4Si$ 470.2, observed LCMS m/z 477.2 (M+H).

Part G:

A solution of compound 60 (40 mg, 0.084 mmol) in DMF (5 mL) was heated at 130 °C for 7 hours. LC-MS analysis of the reaction confirmed product formation but also hydrolysis of the *tert*-butyldimethylsilyl protecting group. Imidazole (17 mg, 0.25 mmol) and *tert*-butyldimethylsilyl chloride (25 mg, 0.17 mmol) was added and the reaction mixture stirred at room temperature for 18 hours. Water (10 mL) was added and the crude product extracted with EtOAc (2 x 10 mL). Drying over magnesium sulfate and concentration afforded crude compound 61 which was further purified by flash column chromatography, gradient elution (0 to 100 %) hexane / ethyl acetate (25 mg, 66 %). HPLC-MS $t_R$ = 2.30 min ($UV_{254\ nm}$); mass calculated for formula $C_{19}H_{29}BrN_2O_4Si$ 456.1, observed LCMS m/z 457.1 (M+H).

Part H:

Compound 62 was prepared from the reaction of compound 61 (40 mg, 0.09 mmol) with phenyl acetylene (18 mg, 0.18 mmol) using the Sonagashira coupling conditions described in Example 8A, Part B. HPLC-MS $t_R$ = 2.61 min (($UV_{254\ nm}$); mass calculated for formula $C_{27}H_{34}N_2O_4Si$ 478.3, observed LCMS m/z 479.2 (M+H).

Part I:

A solution containing compound 62 (21.5 mg, 0.045 mmol) and tetrabutylammonium fluoride (1*M*, 45 μL, 0.045 mmol) in THF (2 mL) was stirred at room temperature for 1 hour. LC-MS analysis indicated that the hydrolysis was complete. The reaction mixture was quenched with the addition of saturated $NH_4Cl$ and extracted with EtOAc (2 x 5 mL). Drying over magnesium sulfate and concentration afforded crude compound 63 which was subjected to flash silica chromatography, gradient elution (0 to 20 %) ethyl acetate / methanol (10 mg, 61 %). HPLC-MS $t_R$ = 1.89 min (($UV_{254\ nm}$); mass calculated for formula $C_{21}\ H_{20}N_2O_4$ 364.1, observed LCMS m/z 365.2 (M+H).

Part J:

Compound 64 (9 mg, 100 %) was prepared from compound 63 (10 mg, 0.027 mmol) using the saponification conditions described in Example 4, Part C. HPLC-MS $t_R$ = 1.49 min ($UV_{254\ nm}$); mass calculated for formula $C_{19}H_{16}N_2O_4$ 336.1, observed LCMS m/z 337.1 (M+H).

Part K:

Compound 65 was prepared from compound 64 using the peptide coupling conditions described in Example 2, Part D.

The following compound, 65 was synthesized using the procedure described 10

**Table-7**

| Cmpd # | Structure | Exact mass | MS m/z ($M^+$+H) | Ret. Time (min) |
|---|---|---|---|---|
| 65 | 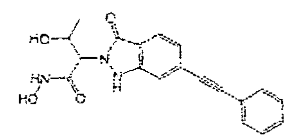 | 351.1 | 352.1 | 3.19 |

**Example 11:**

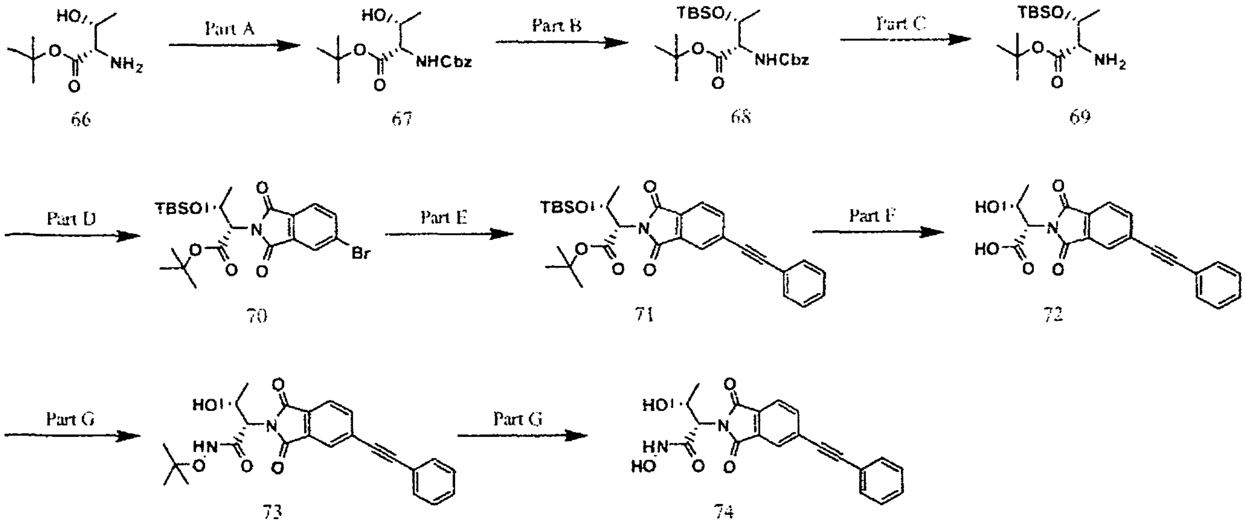


66 67 68 69 70 71 72 73 74

Part A:

To an ice-cooled solution of L-threonine *tert*-butyl ester hydrochloride (66) (2.12 g, 10 mmol) and DIEA (3.83 mL, 22 mmol) in THF (20 mL) was slowly added over 5 minutes a solution of benzyl chloroformate (1.55 mL, 11 mmol) in THF (10 mL). The reaction mixture was warmed to room temperature and stirred for 2 hours. LC-MS analysis indicated the reaction was complete. The reaction was quenched with the addition of 1 *N* HCl and extracted with EtOAc. Drying over magnesium sulfate and concentration afforded crude compound 67 as a yellow oil (2.67 g, 100 %).

Part B:

A solution of compound 67 (653 mg, 2.1 mmol), imidazole (173 mg, 2.5 mmol) and *tert*-butyldimethylsilyl chloride (350 mg, 2.3 mmol) in DMF (10 mL) was stirred at room temperature for 18 hours. LC-MS analysis indicated the reaction was complete. The reaction was quenched with water and extracted with EtOAc. Drying over magnesium sulfate and concentration afforded crude compound 68 as a colorless oil (738 mg, 83 %). HPLC-MS $t_R$ = 2.73 min ($UV_{254\ nm}$); mass calculated for formula $C_{22}H_{37}NO_5Si$ 423.2, observed LCMS m/z 446.1 (M+Na).

Part C:

A solution of compound 68 (738 mg, 1.74 mmol) and palladium on charcoal (10 %) in EtOAc (20 mL) was subjected to hydrogenation for 18 hours. LC-MS analysis indicated the reaction was complete. The reaction mixture was filtered by passing through celite, and evaporated to afford crude compound 69 as a colorless oil (488 mg, 97 %).

HPLC-MS $t_R$ = 1.36 min ($UV_{254\ nm}$); mass calculated for formula $C_{14}H_{31}NO_3Si$ 289.2, observed LCMS m/z 290.3 (M+H).

Part D:

A mixture of compound 69 (145 mg, 0.5 mmol), DIEA (174 □L, 1.0 mmol) and 4-bromophthalic anhydride (170 mg, 0.75 mmol) in dioxane (2 mL) was heated in the microwave for 10 minutes at 160 °C. LC-MS analysis indicated the reaction was complete. The volatiles were removed *in vacuo* and the crude residue subjected to flash silica chromatography, gradient elution (0 to 100 %) hexane / ethyl acetate to afford compound 70 as a colorless oil (101 mg, 41 %). HPLC-MS $t_R$ = 2.76 min ($UV_{254\ nm}$); mass calculated for formula $C_{22}H_{32}BrNO_5Si$ 497.1, observed LCMS m/z 498.1 (M+H).

Part E:

Compound 71 (50 mg, 48 %) was prepared from the reaction of compound 70 (100 mg, 0.2 mmol) with phenylacetylene (41 mg, 0.4 mmol) using the Sonagashira coupling conditions described in Example 8A, Part B. HPLC-MS $t_R$ = 2.94 min ($UV_{254\ nm}$); mass calculated for formula $C_{30}H_{37}NO_5Si$ 519.2, observed LCMS m/z 464.2 (M+H-$^tBu$).

Part F:

Trifluoroacetic acid (2 mL) was added to a stirring solution of compound 71 (71 mg, 0.096 mmol) in DCM (2 mL) and the resulting mixture stirred at room temperature for 10 minutes. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 72. HPLC-MS $t_R$ = 1.72 min ($UV_{254\ nm}$); mass calculated for formula $C_{20}H_{15}NO_5$ 349.1, observed LCMS m/z 350.1 (M+H).

Part G:

Compound 73 was prepared from the reaction of compound 72 (15 mg, 0.043 mmol) and *tert*-butyl-O-hydroxylamine (11 mg, 0.086 mmol) using the peptide coupling conditions described in Example 2, Part D. HPLC-MS $t_R$ = 2.0 min ($UV_{254\ nm}$); mass calculated for formula $C_{24}H_{24}N_2O_5$ 420.2, observed LCMS m/z 421.2 (M+H).

Part H:

Trifluoroacetic acid (2 mL) was added to compound 73 (10 mg, 0.024 mmol) and the resulting mixture stirred at room temperature for 8 hours. LC-MS analysis indicated hydrolysis was complete. The volatiles were removed *in vacuo* and the resulting residue purified by Prep.HPLC to afford compound 74 (1.2 mg, 14 %) as an off white solid.
The following compound, 74 was synthesized using the procedure described in example 11

**Table-8**

| Cmpd # | Structure | Exact mass | MS m/z ($M^+$+H) | Ret. Time (min) |
|---|---|---|---|---|
| 74 | 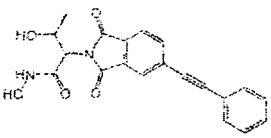 | 364.1 | 365.1 | 3.70 |

**Example 12:**

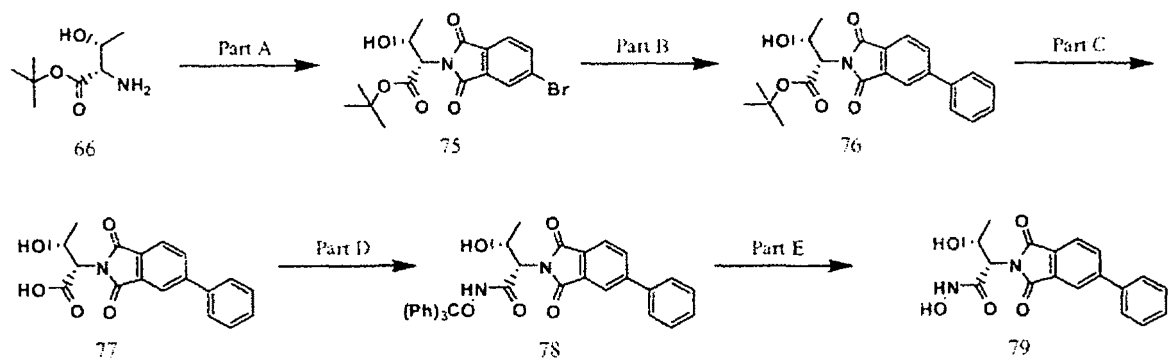


Part A:

Compound 75 (160 mg, 42 %) was prepared from the reaction of L-threonine *tert*-butyl ester hydrochloride (66) (211 mg, 1 mmol) and 4-bromophthalic anhydride (341 mg, 1.5 mmol) using the condensation conditions described in Example 11, Part D. HPLC-MS $t_R$ = 1.85 min ($UV_{254\ nm}$); mass calculated for formula $C_{16}H_{18}BrNO_5$ 383.0, observed LCMS m/z 384.0 (M+H).

Part B:

Compound 76 (40 mg, 70 %) was prepared from the reaction of compound 75 (56 mg, 0.15 mmol) and phenylboronic acid (21 mg, 0.18 mmol) using the Suzuki coupling conditions described in Example 4, Part B. HPLC-MS $t_R$ = 2.15 min ($UV_{254\ nm}$); mass calculated for formula $C_{22}H_{23}NO_5$ 381.2, observed LCMS m/z 326.2 (M+H-$^t$Bu).

Part C:

Compound 77 (75 mg, 100 %) was prepared from compound 76 (90 mg, 0.23 mmol) using the hydrolysis conditions described in Example 11, Part F. HPLC-MS $t_R$ = 1.24 min ($UV_{254\ nm}$); mass calculated for formula $C_{12}H_{10}BrNO_5$ 327.0, observed LCMS m/z 328.0 (M+H).

Part D:

Compound 78 was prepared from the reaction of compound 77 (40 mg, 0.12 mmol) and trityl-O-hydroxylamine (41 mg, 0.14 mmol) using the peptide coupling conditions described in Example 2, Part D. HPLC-MS $t_R$ = 2.49 min ($UV_{254\ nm}$); mass calculated for formula $C_{37}H_{30}N_2O_5$ 582.2, observed LCMS m/z 583.2 (M+H).

Part H:

Trifluoroacetic acid (2 mL) was added to compound 78 (15 mg, 0.025 mmol) and the resulting mixture stirred at room temperature for 1 minute. LC-MS analysis indicated hydrolysis was complete. The volatiles were removed *in vacuo* and the resulting residue purified by Prep.HPLC to afford compound 79 (1.8 mg, 21 %) as an off white solid.

The following compounds 79 and 80 (Table-9) were synthesized using this procedure example 12

**Table- 9**

| Cmpd # | Structure | Exact mass | MS m/z ($M^+$+H) | Ret. Time (min) |
|---|---|---|---|---|
| 79 | 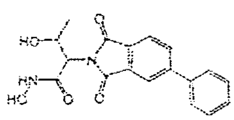 | 340.1 | 341.1 | 3.11 |
| 80 | 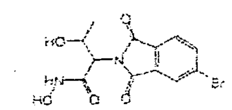 | 342.0 | 343.0 | 2.56 |

**Example 13:**

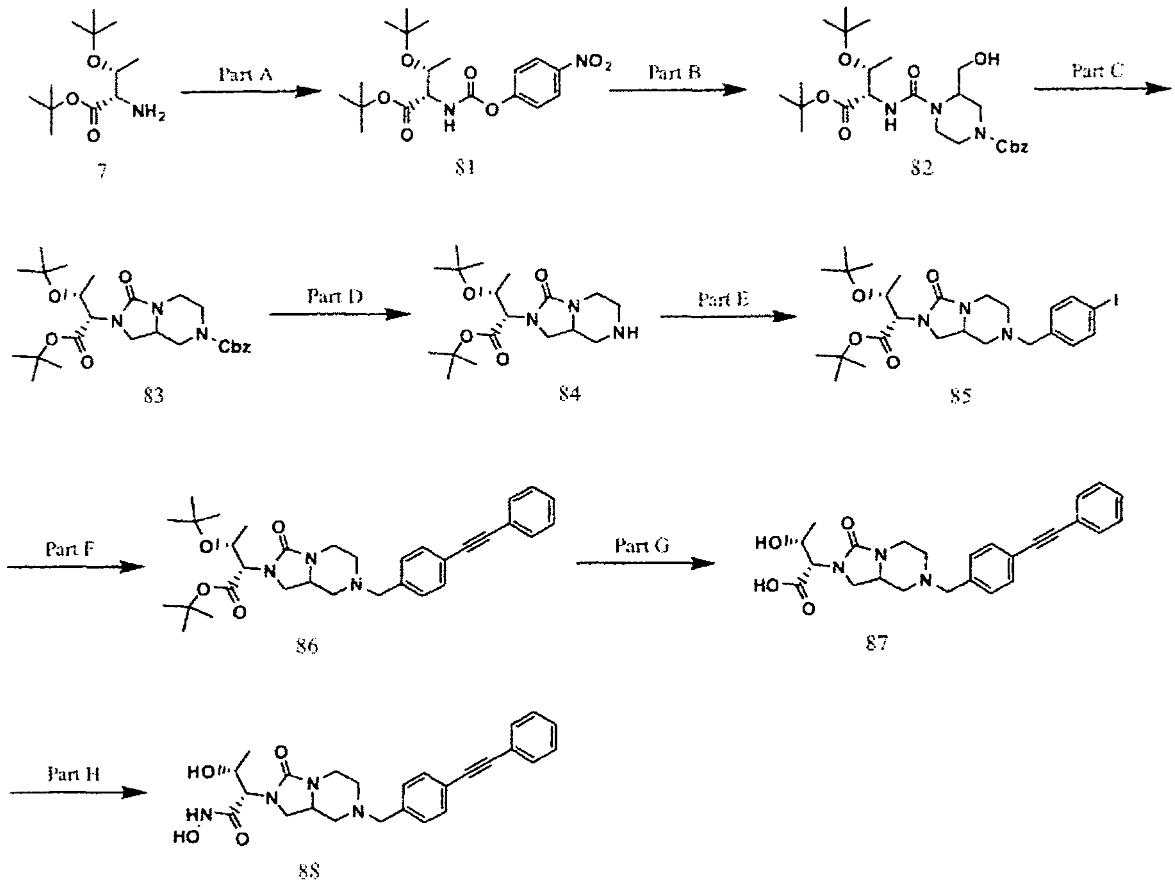


7, 81, 82, 83, 84, 85, 86, 87, 88

Part A:

To an ice-cooled solution of 4-nitrophenyl chloroformate (665 mg, 3.3 mmol) and DIEA (1.6 mL, 9 mmol) in THF (10 mL) was slowly added over 20 minutes a solution of O-*tert*-butyl-L-threonine *tert*-butyl ester hydrochloride (7) (803 mg, 3 mmol) in THF (5 mL). The reaction mixture was warmed to room temperature and stirred for 18 hours. LC-MS analysis indicated the reaction was complete. The reaction was quenched with the addition of saturated $NaHCO_3$ and extracted with EtOAc. Drying over magnesium sulfate and concentration afforded compound 81 which was subjected to flash silica chromatography, gradient elution (0 to 100 %) hexane / ethyl acetate (917 mg, 77 %). HPLC-MS $t_R$ = 2.22 min ($UV_{254\ nm}$); mass calculated for formula $C_{19}H_{28}N_2O_7$ 396.2, observed LCMS m/z 397.1 (M+H).

Part B:

To a solution of compound 81 (396 mg, 1.0 mmol) and DIEA (0.523 □L, 3.0 mmol) in THF (10 mL) was added 4-*N*-benzyloxycarbonyl-2-hydroxymethylpiperazine (300 mg, 1.2 mmol) and the reaction mixture heated at 80 °C for 18 hours. The reaction was quenched with the addition of 1 N HCl and extracted with EtOAc. Drying over magnesium sulfate and concentration afforded compound 82 which was subjected to flash silica chromatography, gradient elution (0 to 100 %) hexane / ethyl acetate (345 mg, 68 %). HPLC-MS $t_R$ = 1.99 min ($UV_{254\ nm}$); mass calculated for formula $C_{26}H_{41}N_3O_7$ 507.3, observed LCMS m/z 508.3 (M+H).

Part C:

Methanesulfonyl chloride (61 μL, 0.79 mmol) was added to an ice-cooled solution of compound 82 (334 mg, 0.66 mmol) in DCM (6 mL) and pyridine (3 mL). The reaction mixture was stirred at 0 °C for 1 hour and then warmed to room temperature. LC-MS analysis indicated the reaction was complete. The reaction was quenched with the addition

of 1*N* HCl and extracted with EtOAc. Drying over magnesium sulfate and concentration afforded compound 83 which was subjected to flash silica chromatography, gradient elution (0 to 100 %) hexane / ethyl acetate (290 mg, 90 %). HPLC-MS $t_R$ = 1.39 min ($UV_{254\ nm}$); mass calculated for formula $C_{26}H_{39}N_3O_6$ 489.3, observed LCMS m/z 490.3 (M+H).

Part D:

Compound 84 (210 mg, 100 %) was prepared from compound 73 (290 mg, 0.59 mmol) using the hydrogenation conditions described in Example 3, Part B. HPLC-MS $t_R$ = 1.19 min ($UV_{254\ nm}$); mass calculated for formula $C_{18}H_{33}N_3O_4$ 355.2, observed LCMS m/z 356.3 (M+H).

Part E:

A mixture of compound 84 (112 mg, 0.32 mmol), potassium carbonate (52 mg, 0.38 mmol) and 4-iodobenzyl bromide (112 mg, 0.38 mmol) in DMF (5 mL) was heated at 60 °C for 3 hours. LC-MS analysis indicated the reaction was complete. The reaction was quenched with the addition of saturated $NaHCO_3$ and extracted with EtOAc.

Drying over magnesium sulfate and concentration afforded compound 85 which was subjected to flash silica chromatography, gradient elution (0 to 100 %) hexane / ethyl acetate (25 mg, 14 %). HPLC-MS $t_R$ = 1.56 min ($UV_{254\ nm}$); mass calculated for formula $C_{25}H_{38}IN_3O_4$ 571.2, observed LCMS m/z 572.2 (M+H).

Part F:

To a mixture of compound 85 (25 mg, 0.043 mmol), copper iodide (0.5 mg, 2.58 μmol) and dichlorobis(triphenylphosphine)palladium (II) (1.1 mg, 1.5 μmol) in THF (2 mL) was added phenylacetylene (7 mg, 0.065 mmol) and triethylamine (14 μL, 0.1 mmol). The reaction vessel was flushed with argon, and the reaction mixture stirred at room temperature for 18 hours. LC-MS analysis of the reaction indicated that the reaction was complete. Ethyl acetate (5 mL) was added and the reaction mixture washed with saturated $NaHCO_3$. Drying over magnesium sulfate, concentration and purification by flash column chromatography, gradient elution (0 to 100 %) hexane / ethyl acetate, afforded compound 86 as a yellow solid (23 mg, 98 % yield). HPLC-MS $t_R$ = 1.97 min ($UV_{254\ nm}$); mass calculated for formula $C_{33}H_{43}N_3O_4$ 545.3, observed LCMS m/z 546.3 (M+H).

Part G:

Trifluoroacetic acid (3 mL) was added to compound 86 (23 mg, 0.42 mmol) and the resulting mixture stirred at room temperature for 1 hour. LC-MS analysis indicated hydrolysis was complete. The volatiles were removed *in vacuo* and the resulting residue re-dissolved in a 1:1 MeCN / water mixture (10 mL) and lyophilized for 18 hours to afford crude compound 87. HPLC-MS $t_R$ = 1.50 min ($UV_{254\ nm}$); mass calculated for formula $C_{25}H_{27}N_3O_4$ 433.2, observed LCMS m/z 434.2 (M+H).

Part H:

Compound 88 was prepared from compound 87 using the peptide coupling conditions described in Example 2, Part D.

The following compound, 88 was synthesized using the procedure described in example 13:

**Table-10**

| Compound # | Structure | Exact mass | MS m/z ($M^{+}$+H) | Ret. Time (min) |
|---|---|---|---|---|
| 88 | 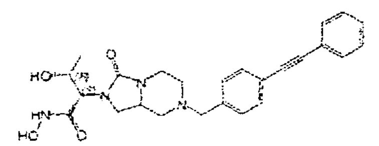 | 448.2 | 449.2 | 2.93 |

**Example 14:**

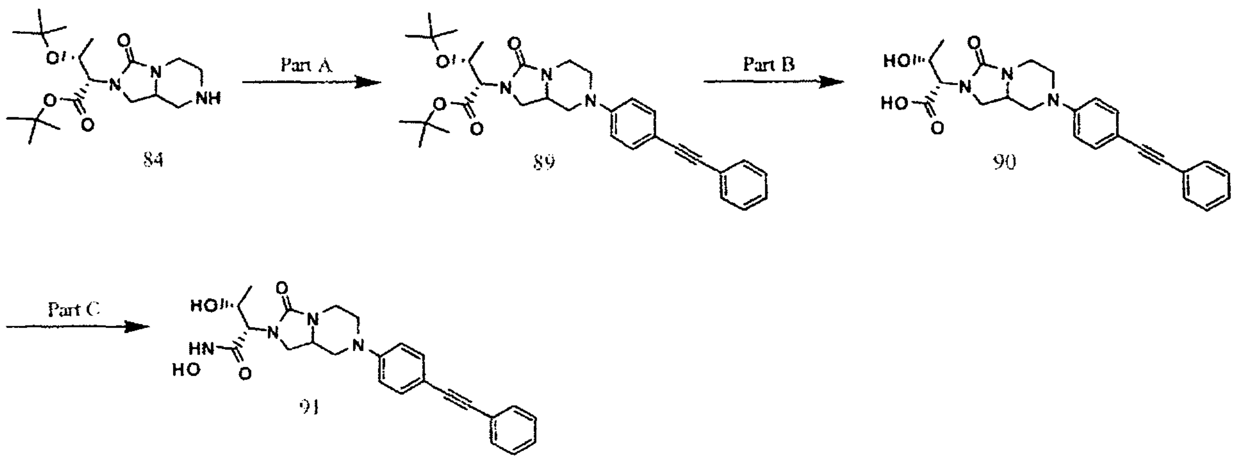


84 89 90 91

Part A:

Compound 89 (40 mg, 23 %) was prepared from the reaction of compound 84 (118 mg, 0.33 mmol) and 1-bromo-4-(phenylethynyl)benzene using the coupling conditions described in Example 9, Part A. HPLC-MS $t_R$ = 1.89 min ($UV_{254\ nm}$); mass calculated for formula $C_{32}H_{41}N_3O_4$ 531.3, observed LCMS m/z 532.3 (M+H).

Part B:

Trifluoroacetic acid (3 mL) was added to compound 89 (25 mg, 0.047 mmol) and the resulting mixture stirred at room temperature for 1 hour. LC-MS analysis indicated hydrolysis was complete. The volatiles were removed *in vacuo* and the resulting residue re-dissolved in a 1:1 MeCN /water mixture (10 mL) and lyophilized for 18 hours to afford crude compound 90. HPLC-MS $t_R$ = 1.55 min ($UV_{254\ nm}$); mass calculated for formula $C_{24}H_{25}N_3O_4$ 419.2, observed LCMS m/z 420.2 (M+H).

Part H:

Compound 91 was prepared from compound 90 using the peptide coupling conditions described in Example 2, Part D.

The following compound, 91 was synthesized using the procedure in example 14:

**Table-11**

| Compound # | Structure | Exact mass | MS m/z ($M^+$+H) | Ret. Time (min) |
|---|---|---|---|---|
| 91 | 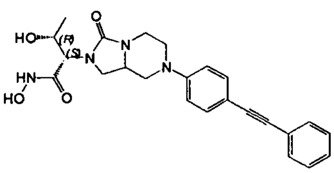 | 434.2 | 435.2 | 3.44 |

**Claims**

**1.** A compound having the formula:

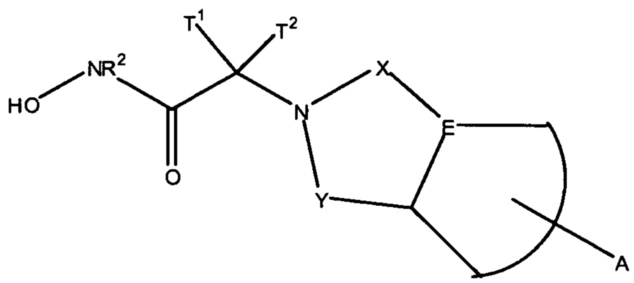

(I)

and pharmaceutically acceptable salts, solvates and esters thereof, wherein:

$T^1$ is H and $T^2$ is ethyl or isopropyl, wherein said ethyl or isopropyl is substituted with -OH or $NH_2$;
X is C(O);
Y is C(O) or $CH_2$;
E is C, CH, or N;

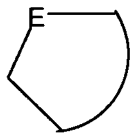

is a six-membered ring selected from the group consisting of aryl, cycloalkenyl, cycloalkyl, heteroaryl, heterocyclenyl and heterocyclyl;

A is selected from the group consisting of ethynyl, phenyl, phenylmethyl, piperidine, piperazine, bromo and chloro, wherein said ethynyl, phenyl, phenylmethyl, piperidine, and piperazine can be unsubstituted or substituted with an additional phenyl or additional ethynyl: wherein said additional phenyl can be unsubstituted or substituted with another phenyl or ethynyl, still further wherein, said another phenyl, can be unsubstituted or substituted with still another phenyl and; wherein said additional ethynyl is substituted with another phenyl,
wherein said another phenyl can be unsubstituted or substituted with still another phenyl;
or A is

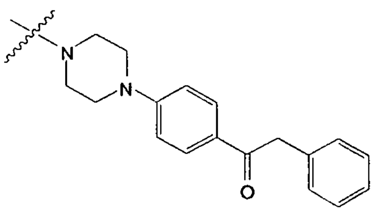

;

$R^2$ is hydrogen;

**2.** The compound according to claim 1, wherein

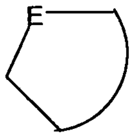

is aryl or heterocyclyl.

3. The compound according to claim 2, wherein said aryl is phenyl or said heterocyclyl is piperazine.

4. The compound of claim 1, 2 or 3, wherein A is alkynyl with two to fifteen carbon atoms substituted with phenyl, wherein said phenyl can be unsubstituted or further substituted with an additional phenyl.

5. The compound of claim 4, wherein said alkynyl is ethynyl.

6. The compound of claim 5, wherein said ethynyl is substituted with phenyl, which is meta or para substituted with said additional phenyl.

7. The compound of claim 1, 2 or 3, wherein A is phenyl, which can be unsubstituted or substituted with alkynyl with two to fifteen carbon atoms, wherein said alkynyl is substituted with phenyl; or A is phenyl, substituted with phenyl preferably in the para position; or A is piperidinyl, substituted with phenyl.

8. The compound of claim 7, wherein said alkynyl is ethynyl.

9. The compound of claim 7 wherein said piperidinyl is para substituted with phenyl substituted with phenyl; or wherein said piperidinyl is para substituted with phenyl, wherein said phenyl is para substituted with an additional phenyl; or wherein said phenyl substituting said piperidinyl is substituted with alkynyl with two to fifteen carbon atoms, wherein said alkynyl is substituted with phenyl; or wherein said piperidinyl is para substituted with phenyl, wherein said phenyl is substituted with ethynyl, which is substituted with phenyl.

10. A compound of claim 1 selected from:

| | |
|---|---|
| 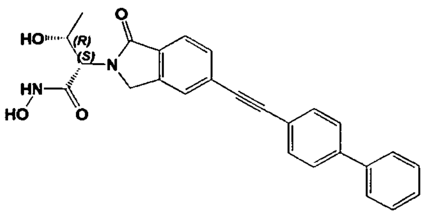 | 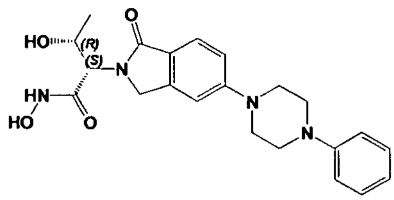 |

| | |
|---|---|
| 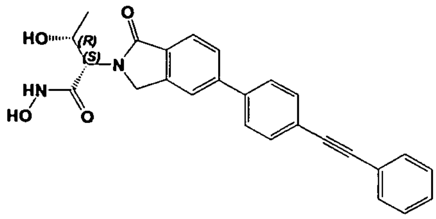 | 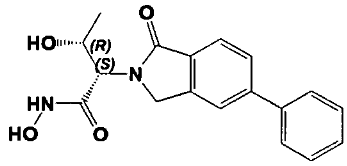 |
| 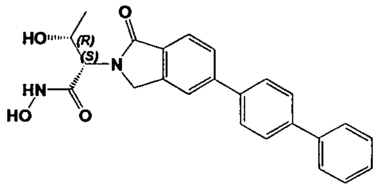 | 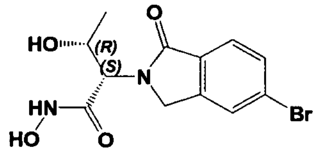 |
| 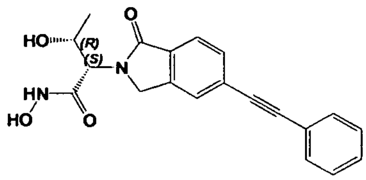 | 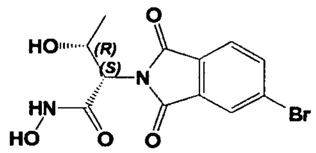 |
| 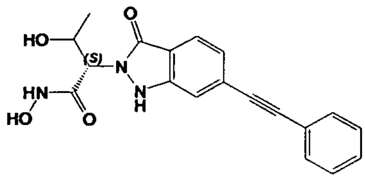 | 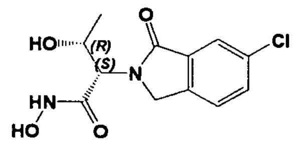 |
| 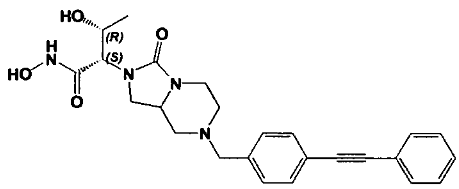 | 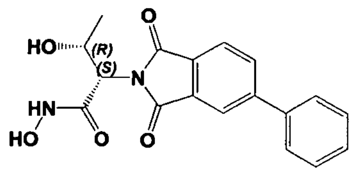 |
| 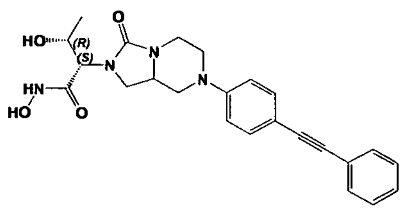 | 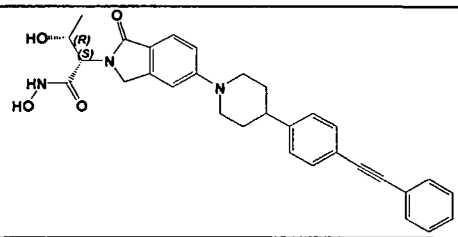 |
| 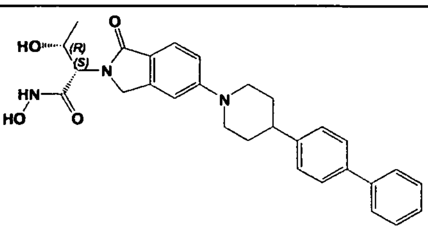 | 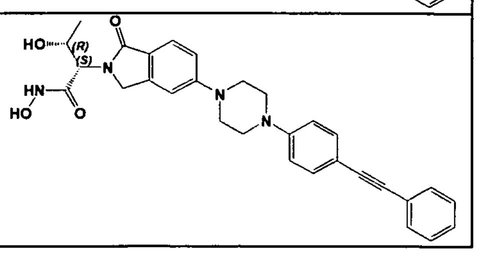 |

| | |
|---|---|
| 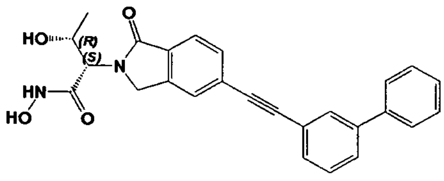 | 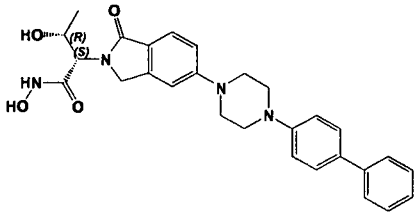 |
| 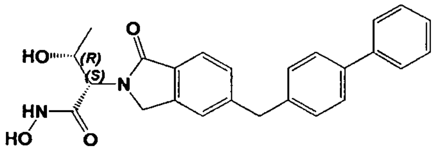 | 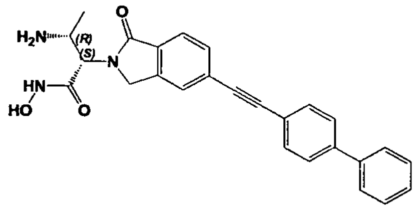 |
| 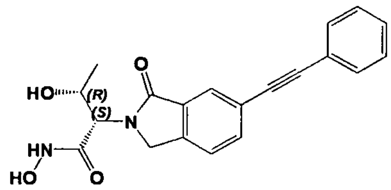 | 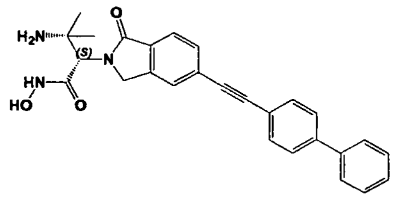 |
| 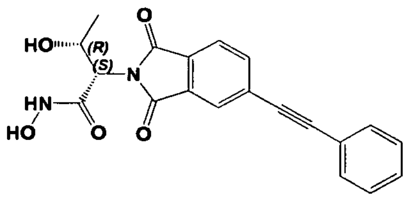 and | 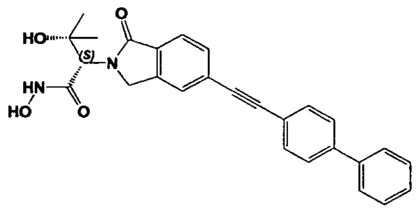 |

or a pharmaceutically acceptable salt, solvate or ester thereof.

**11.** A compound according to any preceding claim, in purified form.

**12.** A pharmaceutical composition comprising at least one compound of any one of claims 1 to 10, or a pharmaceutically acceptable salt, solvate or ester thereof, in combination with at least one pharmaceutically acceptable carrier.

**13.** A combination of a compound of any one of claims 1 to 10, or a pharmaceutically acceptable salt, solvate or ester thereof, and at least one additional agent, drug, medicament, antibody and/or inhibitor for treating a UDP-3-*O*-(R-3-hydroxymyristoyl)-N-acetylglucosamine deacetylase (LpxC) receptor mediated disease or an antibacterial agent.

**14.** A compound of any one of claims 1 to 10, or a pharmaceutically acceptable salt, solvate or ester thereof, for use in a treatment of the human body by therapy.

**15.** The compound for use of claim 14 for the treatment of a microbial infection.

**16.** The compound for use of claim 15, wherein said microbial infection is a bacterial or fungal infection

**17.** The compound for use of claim 16, wherein said bacterial infection is a gram negative infection or a gram positive infection.

**18.** The compound for use of claim 15, wherein said microbial infection is caused by at least one organism selected from the group consisting *of Acinetobacter baumannii, Acinetobacter calcoaceticus, Acinetobacter haemolyticus, Acinetobacter hydrophila, Actinobacillus actinomycetemcomitans, Aeromonas hydrophila, Alcaligenes xylosoxidans, Bacteroides distasonis, Bacteroides fragilis, Bacteroides melaninogenicus, Bacteroides ovatus, Bacteroides thetaiotaomicron, Bacteroides vulgatus, Bartonella henselae, Bordetella pertussis, Branhamella catarrhalis, Brucella*

*melitensis, Brucella abortus, Brucella canis, Burkholderia cepacia, Burkholderia mallei, Burkholderia pseudomallei, Campylobacter coli, Campylobacter fetus, Campylobacter jejuni, Citrobacter diversus, Citrobacter freundii, Citrobacter koseri, Coxiella burnetli, Edwarsiella tarda, Ehrlichia chafeenis, Eikenella corrondens, Enterobacter aerogenes, Enterobacter agglomerans, Enterobacter cloacae, Escherichia coli, Flavobacterium meningosepticum, Francisella tularensis, Fusobacterium spp., Haemophilus ducreyi, Haemophilus influenzae, Haemophilus parainfluenzae, Helicobacter pylori, Kingella kingae, Klebsiella oxytoca, Klebsiella ozaenae, Klebsiella pneumoniae, Klebsiella rhinoscleromatis, Legionella pneumophila, Moraxella catarrhalis, Morganella morganii, Neisseria gonorrhoeae, Neisseria meningitides, Pasteurella multocida, Plesiomonas shigelloides, Porphyromonas asaccharolytica, Porphyromonas gingivalis, Prevotella bivia, Prevotella buccae, Prevotella corporis, Prevotella endodontalis, Prevotella intermedia, Prevotella melaninogenica, Prevotella oralis, Proteus mirabilis, Proteus myxofaciens, Proteus penner, Proteus vulgaris, Providencia alcalifaciens, Providencia rettgeri, Providencia stuarfii, Pseudomonas aeruginosa, Pseudomonas fluorescens, Ricketsia prowozekii, Salmonella enterica, Serratia marcescens, Shigella boydii, Shigella dysenteriae, Shigella flexneri, Shigella sonnei, Stenotrophomonas maltophilia, Streptobacillus moniliformis, Vibrio alginolyticus, Vibrio cholerae, Vibrio parahaemolyticus, Vibrio vuluificus, Yersinia enterocolitica, Yersinia pestis,* and *Yersinia pseudotuberculosis.*

**19.** Use of a compound of any one of claims 1 to 10, or a pharmaceutically acceptable salt, solvate or ester thereof for the manufacture of a medicament for treating a condition as defined in any one of claims 15 to 18.

**Patentansprüche**

**1.** Eine Verbindung der Formel:

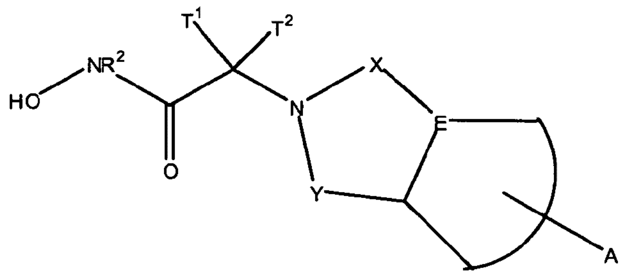

und pharmazeutisch annehmbare Salze, Solvate und Ester davon,
wobei:

$T^1$ H ist und $T^2$ Ethyl oder Isopropyl ist, wobei das Ethyl oder Isopropyl substituiert ist mit -OH oder $NH_2$,
X C(O) ist,
Y C(O) oder $CH_2$ ist,
E C, CH oder N ist,

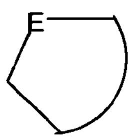

ein sechsgliedriger Ring ist, ausgewählt aus der Gruppe, bestehend aus Aryl, Cycloalkenyl, Cycloalkyl, Heteroaryl, Heterocyclenyl und Heterocyclyl,
A ausgewählt ist aus der Gruppe, bestehend aus Ethinyl, Phenyl, Phenylmethyl, Piperidin, Piperazin, Brom und Chlor, wobei das Ethinyl, Phenyl, Phenylmethyl, Piperidin und Piperazin unsubstituiert oder substituiert sein kann mit einem zusätzlichen Phenyl oder zusätzlichem Ethinyl, wobei das zusätzliche Phenyl unsubstituiert oder substituiert sein kann mit einem weiteren Phenyl oder Ethinyl, wobei ferner das weitere Phenyl unsubstituiert oder substituiert sein kann mit noch einem weiteren Phenyl, und wobei das zusätzliche Ethinyl substituiert ist mit einem weiteren Phenyl, wobei das weitere Phenyl unsubstituiert oder substituiert sein kann mit noch einem weiteren Phenyl,

oder A

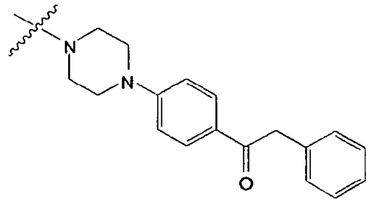

ist,

$R^2$ Wasserstoff ist.

**2.** Die Verbindung gemäß Anspruch 1, wobei

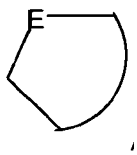

Aryl oder Heterocyclyl ist.

**3.** Die Verbindung gemäß Anspruch 2, wobei das Aryl Phenyl ist oder das Heterocyclyl Piperazin ist.

**4.** Die Verbindung nach Anspruch 1, 2 oder 3, wobei A Alkinyl mit zwei bis fünfzehn Kohlenstoffatomen ist, substituiert mit Phenyl, wobei das Phenyl unsubstituiert oder weiter substituiert sein kann mit einem zusätzlichen Phenyl.

**5.** Die Verbindung nach Anspruch 4, wobei das Alkinyl Ethinyl ist.

**6.** Die Verbindung nach Anspruch 5, wobei das Ethinyl substituiert ist mit Phenyl, das meta-oder para-substituiert ist mit dem zusätzlichen Phenyl.

**7.** Die Verbindung nach Anspruch 1, 2 oder 3, wobei A Phenyl ist, das unsubstituiert oder substituiert sein kann mit Alkinyl mit zwei bis fünfzehn Kohlenstoffatomen, wobei das Alkinyl substituiert ist mit Phenyl, oder A Phenyl ist, substituiert mit Phenyl vorzugsweise in der para-Position, oder A Piperidinyl ist, substituiert mit Phenyl.

**8.** Die Verbindung nach Anspruch 7, wobei das Alkinyl Ethinyl ist.

**9.** Die Verbindung nach Anspruch 7, wobei das Piperidinyl para-substituiert ist mit Phenyl, das mit Phenyl substituiert ist, oder wobei das Piperidinyl para-substituiert ist mit Phenyl, wobei das Phenyl para-substituiert ist mit einem zusätzlichen Phenyl, oder wobei das Phenyl als Substituent des Piperidinyl substituiert ist mit Alkinyl mit zwei bis fünfzehn Kohlenstoffatomen, wobei das Alkinyl substituiert ist mit Phenyl, oder wobei das Piperidinyl para-substituiert ist mit Phenyl, wobei das Phenyl substituiert ist mit Ethinyl, das mit Phenyl substituiert ist.

**10.** Eine Verbindung nach Anspruch 1, ausgewählt aus:

| | |
|---|---|
| 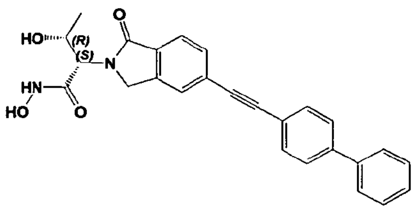 | 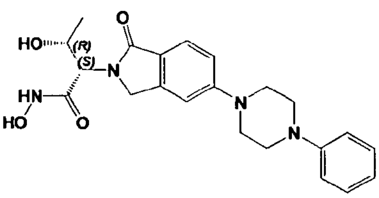 |

| | |
|---|---|
| 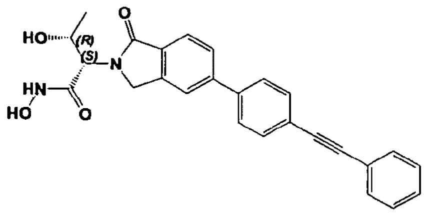 | 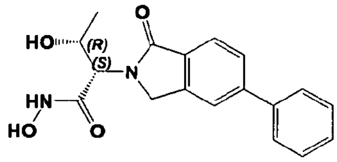 |
| 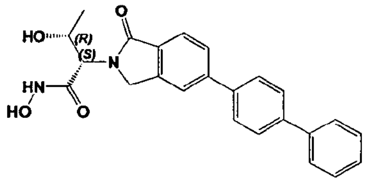 | 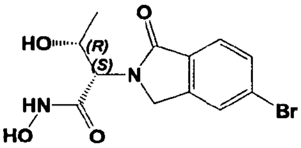 |
| 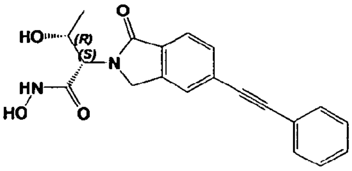 | 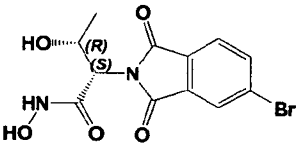 |
| 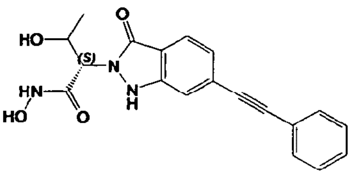 | 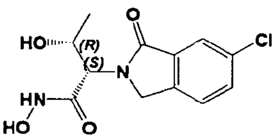 |
| 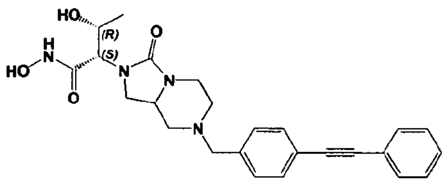 | 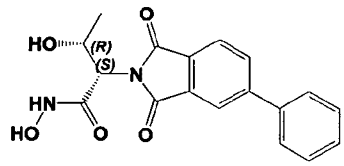 |
| 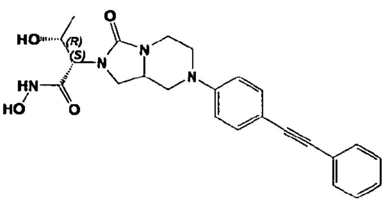 | 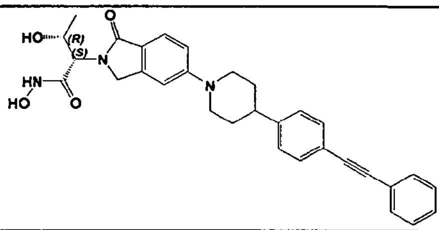 |
| 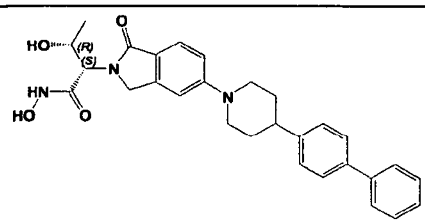 | 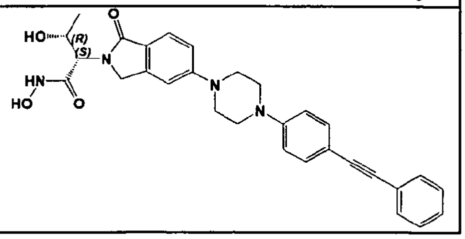 |

| | |
|---|---|
| 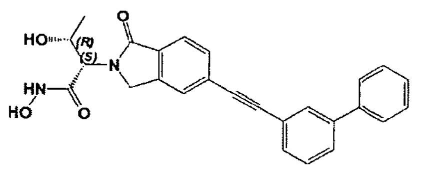 | 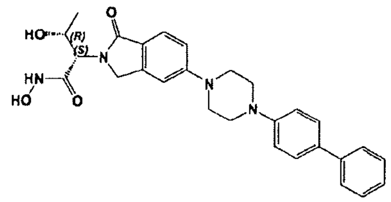 |
| 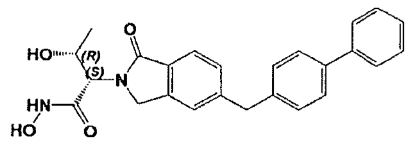 | 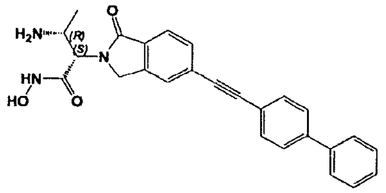 |
| 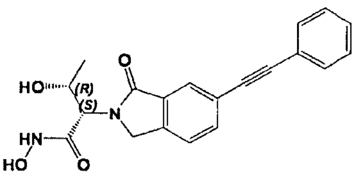 | 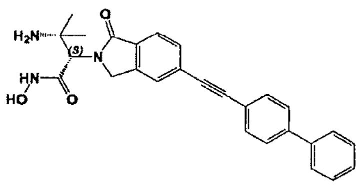 |
| 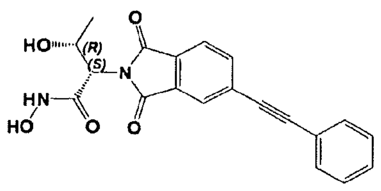 und | 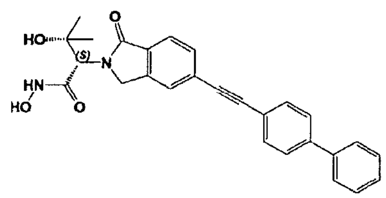 |

oder ein pharmazeutisch annehmbares Salz, Solvat oder Ester davon.

**11.** Eine Verbindung gemäß einem vorhergehenden Anspruch in gereinigter Form.

**12.** Eine pharmazeutische Zusammensetzung, umfassend wenigstens eine Verbindung nach einem der Ansprüche 1 bis 10 oder ein pharmazeutisch annehmbares Salz, ein pharmazeutisch annehmbares Solvat oder ein pharmazeutisch annehmbarer Ester davon in Kombination mit wenigstens einem pharmazeutisch annehmbaren Träger.

**13.** Eine Kombination aus einer Verbindung nach einem der Ansprüche 1 bis 10 oder einem pharmazeutisch annehmbaren Salz, Solvat oder Ester davon und wenigstens einem zusätzlichen Mittel, Arzneistoff, Medikament, Antikörper und/oder Inhibitor zur Behandlung einer UDP-3-O-(R-3-Hydroxymyristoyl)-N-acetylglucosamindeacetylase (LpxC)-Rezeptor-vermittelten Erkrankung oder einem antibakteriellen Mittel.

**14.** Eine Verbindung nach einem der Ansprüche 1 bis 10 oder ein pharmazeutisch annehmbares Salz, ein pharmazeutisch annehmbares Solvat oder ein pharmazeutisch annehmbarer Ester davon zur Verwendung bei einer Behandlung des menschlichen Körpers durch Therapie.

**15.** Die Verbindung zur Verwendung nach Anspruch 14 zur Behandlung einer mikrobiellen Infektion.

**16.** Die Verbindung zur Verwendung nach Anspruch 15, wobei die mikrobielle Infektion eine Bakterien- oder Pilzinfektion ist.

**17.** Die Verbindung zur Verwendung nach Anspruch 16, wobei die Bakterieninfektion eine gramnegative Infektion oder eine grampositive Infektion ist.

**18.** Die Verbindung zur Verwendung nach Anspruch 15, wobei die mikrobielle Infektion durch wenigstens einen Organismus verursacht wird, ausgewählt aus der Gruppe, bestehend aus *Acinetobacter baumannii, Acinetobacter calcoaceticus, Acinetobacter haemolyticus, Acinetobacter hydrophila, Actinobacillus actinomycetemcomitans, Aeromonas hydrophila, Alcaligenes xylosoxidans, Bacteroides distasonis, Bacteroides fragilis, Bacteroides melanino-*

*genicus, Bacteroides ovatus, Bacteroides thetaiotaomicron, Bacteroides vulgatus, Bartonella henselae, Bordetella pertussis, Branhamella catarrhalis, Brucella melitensis, Brucella abortus, Brucella canis, Burkholderia cepacia, Burkholderia mallei, Burkholderia pseudomallei, Campylobacter coli, Campylobacter fetus, Campylobacter jejuni, Citrobacter diversus, Citrobacter freundii, Citrobacter koseri, Coxiella burnetli, Edwarsiella tarda, Ehrlichia chafeenis, Eikenella corrondens, Enterobacter aerogenes, Enterobacter agglomerans, Enterobacter cloacae, Escherichia coli, Flavobacterium meningosepticum, Francisella tularensis, Fusobacterium spp., Haemophilus ducreyi, Haemophilus influenzae, Haemophilus parainfluenzae, Helicobacter pylori, Kingella kingae, Klebsiella oxytoca, Klebsiella ozaenae, Klebsiella pneumoniae, Klebsiella rhinoscleromatis, Legionella pneumophila, Moraxella catarrhalis, Morganella morganii, Neisseria gonorrhoeae, Neisseria meningitides, Pasteurella multocida, Plesiomonas shigelloides, Porphyromonas asaccharolytica, Porphyromonas gingivalis, Prevotella bivia, Prevotella buccae, Prevotella corporis, Prevotella endodontalis, Prevotella intermedia, Prevotella melaninogenica, Prevotella oralis, Proteus mirabilis, Proteus myxofaciens, Proteus penner, Proteus vulgaris, Providencia alcalifaciens, Providencia rettgeri, Providencia stuarfii, Pseudomonas aeruginosa, Pseudomonas fluorescens, Ricketsia prowozekii, Salmonella enterica, Serratia marcescens, Shigella boydii, Shigella dysenteriae, Shigella flexneri, Shigella sonnei, Stenotrophomonas maltophilia, Streptobacillus moniliformis, Vibrio alginolyticus, Vibrio cholerae, Vibrio parahaemolyticus, Vibrio vuluificus, Yersinia enterocolitica, Yersinia pestis* und *Yersinia pseudotuberculosis.*

**19.** Verwendung einer Verbindung nach einem der Ansprüche 1 bis 10 oder eines pharmazeutisch annehmbaren Salzes, Solvats oder Esters davon zur Herstellung eines Medikaments zur Behandlung eines Zustandes wie in einem der Ansprüche 15 bis 18 definiert.

## Revendications

**1.** Composé présentant la formule:

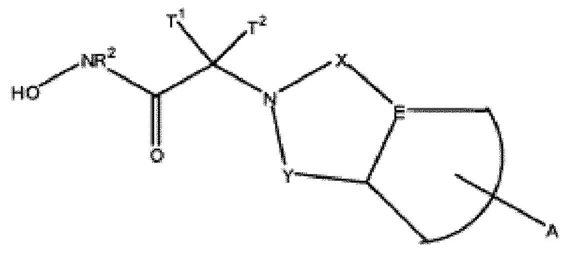

(I)

et sels pharmaceutiquement acceptables, solvates et esters de celui-ci,
dans lequel:

$T^1$ est H et $T^2$ est un éthyle ou un isopropyle, dans lequel ledit éthyle ou isopropyle est substitué avec -OH ou $NH_2$,
X est C(O);
Y est C(O) ou $CH_2$;
E est C, CH ou N;

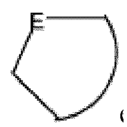

est un cycle de 6 membres choisi dans le groupe constitué d'aryle, de cycloalcényle, de cycloalkyle, d'hétéroaryle, d'hétérocyclényle et d'hétérocyclyle;
A est choisi dans le groupe constitué d'éthynyle, de phényle, de phénylméthyle, de pipéridine, de pipérazine, de bromo et de chloro, dans lequel lesdits éthynyle, phényle, phénylméthyle, pipéridine et pipérazine peuvent être non substitués ou substitués avec un phényle supplémentaire ou un éthynyle supplémentaire; dans lequel ledit phényle supplémentaire peut être non substitué ou substitué avec un autre phényle ou éthynyle, dans lequel encore, ledit autre phényle peut être non substitué ou substitué avec encore un autre phényle; et dans

lequel ledit éthynyle supplémentaire est substitué avec un autre phényle, dans lequel ledit autre phényle peut être non substitué ou substitué avec encore un autre phényle;
ou A est:

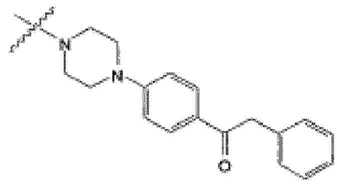

$R^2$ est un hydrogène.

**2.** Composé selon la revendication 1, dans lequel:

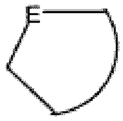

est un aryle ou un hétérocyclyle.

**3.** Composé selon la revendication 2, dans lequel ledit aryle est un phényle et ledit hétérocyclyle est une pipérazine.

**4.** Composé selon la revendication 1, 2 ou 3, dans lequel A est un alcynyle avec de deux à quinze atomes de carbone substitués avec un phényle, dans lequel ledit phényle peut être non substitué ou substitué en plus avec un phényle supplémentaire.

**5.** Composé selon la revendication 4, dans lequel ledit alcynyle est un éthynyle.

**6.** Composé selon la revendication 5, dans lequel ledit éthynyle est substitué avec un phényle, qui est substitué en méta ou en para avec ledit phényle supplémentaire.

**7.** Composé selon la revendication 1, 2 ou 3, dans lequel A est un phényle, qui peut être non substitué ou substitué avec un alcynyle avec de deux à quinze atomes de carbone, dans lequel ledit alcynyle est substitué avec un phényle; ou A est un phényle, substitué avec un phényle de préférence dans la position para; ou A est un pipéridinyle, substitué avec un phényle.

**8.** Composé selon la revendication 7, dans lequel ledit alcynyle est un éthynyle.

**9.** Composé selon la revendication 7, dans lequel ledit pipéridinyle est substitué en para avec un phényle substitué avec un phényle; ou dans lequel ledit pipéridinyle est substitué en para avec un phényle, dans lequel ledit phényle est substitué en para avec un phényle supplémentaire; ou dans lequel ledit phényle substituant ledit pipéridinyle est substitué avec un alcynyle avec de deux à quinze atomes de carbone, dans lequel ledit alcynyle est substitué avec un phényle; ou dans lequel ledit pipéridinyle est substitué en para avec un phényle, dans lequel ledit phényle est substitué avec un éthynyle, qui est substitué avec un phényle.

**10.** Composé selon la revendication 1, choisi parmi:

| | |
|---|---|
| 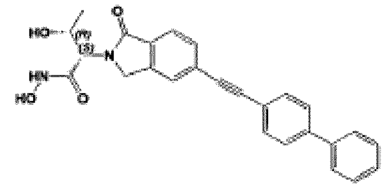 | 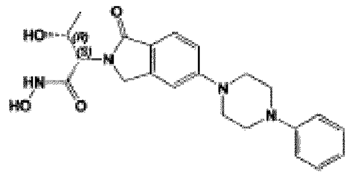 |

| | |
|---|---|
| 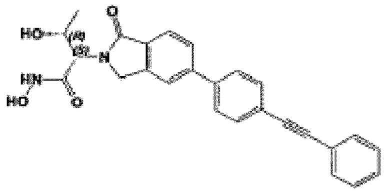 | 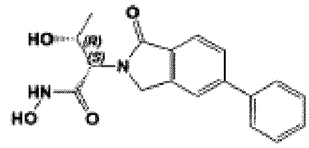 |
| 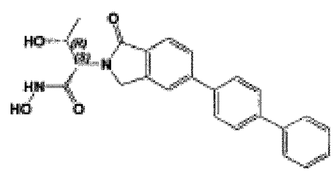 | 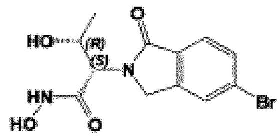 |
| 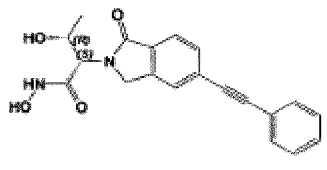 | 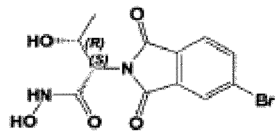 |
| 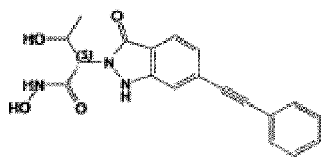 | 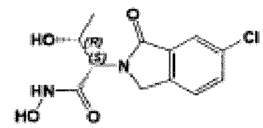 |
| 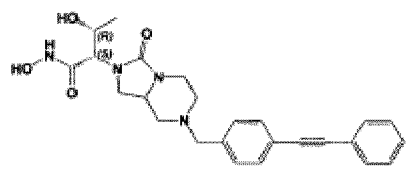 | 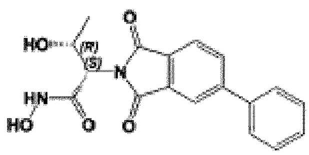 |
| 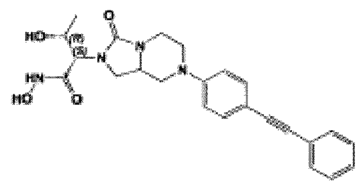 | 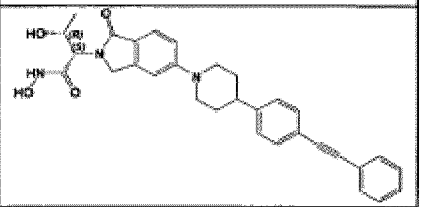 |
| 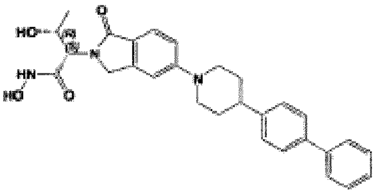 | 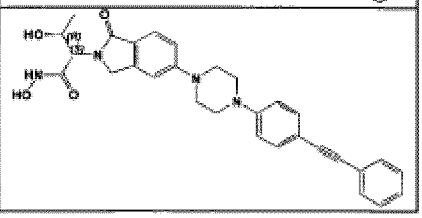 |

| | |
|---|---|
| 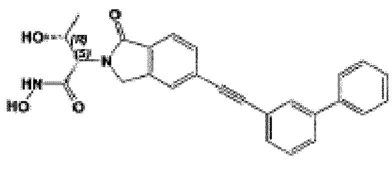 | 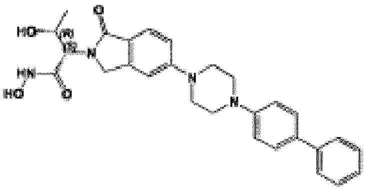 |
| 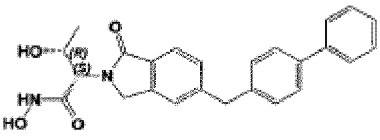 | 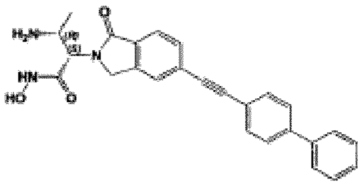 |
| 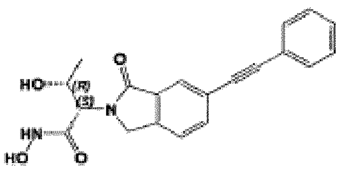 | 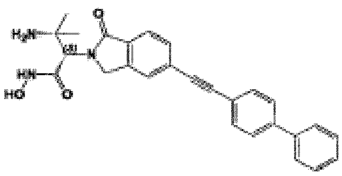 |
| 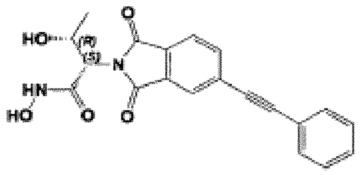 et | 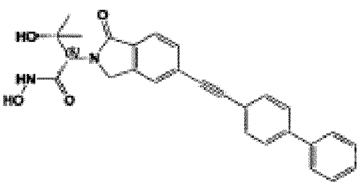 |

ou un sel pharmaceutiquement acceptable, un solvate et un ester de ceux-ci.

**11.** Composé selon l'une quelconque des revendications précédentes, sous une forme purifiée.

**12.** Composition pharmaceutique comprenant au moins un composé selon l'une quelconque des revendications 1 à 10, ou un sel pharmaceutiquement acceptable, un solvate ou un ester de celui-ci, en combinaison avec au moins un support pharmaceutiquement acceptable.

**13.** Combinaison d'un composé selon l'une quelconque des revendications 1 à 10, ou d'un sel pharmaceutiquement acceptable, d'un solvate ou d'un ester de celui-ci, et d'au moins un agent, un produit pharmaceutique, un médicament, un anticorps et/ou un inhibiteur supplémentaire pour le traitement d'une maladie induite par un récepteur d'UDP-3-O-(R-3-hydroxymyristoyl)-N-acétylglucosamine désacétylase (LpxC) ou d'un agent antibactérien.

**14.** Composé selon l'une quelconque des revendications 1 à 10, ou un sel pharmaceutiquement acceptable, un solvate ou un ester de celui-ci, pour une utilisation dans un traitement du corps humain par une thérapie.

**15.** Composé pour une utilisation selon la revendication 14 pour le traitement d'une infection microbienne.

**16.** Composé pour une utilisation selon la revendication 15, dans lequel ladite infection microbienne est une infection bactérienne ou fongique.

**17.** Composé pour une utilisation selon la revendication 16, dans lequel ladite infection bactérienne est une infection gram-négative ou une infection gram-positive.

**18.** Composé pour une utilisation selon la revendication 15, dans lequel ladite infection microbienne est occasionnée par au moins un organisme choisi dans le groupe constitué de: *Acinetobacter baumannii, Acinetobacter calcoaceticus, Acinetobacter haemolyticus, Acinetobacter hydrophila, Actinobacillus actinomycetemcomitans, Aeromonas hydrophila, Alcaligenes xylosoxidans, Bacteroides distasonis, Bacteroides fragilis, Bacteroides melaninogenicus, Bacteroides ovatus, Bacteroides thetaiotaomicron, Bacteroides vulgatus, Bartonella henselae, Bordetella pertussis, Branhamella catarrhalis, Brucella melitensis, Brucella abortus, Brucella canis, Burkholderia cepacia, Burkholderia*

*mallei, Burkholderia pseudomallei, Campylobacter coli, Campylobacter fetus, Campylobacter jejuni, Citrobacter diversus, Citrobacter freundii, Citrobacter koseri, Coxiella burnetli, Edwarsiella tarda, Ehrlichia chafeenis, Eikenella corrondens, Enterobacter aerogenes, Enterobacter agglomerans, Enterobacter cloacae, Escherichia coli, Flavobacterium meningosepticum, Francisella tularensis, Fusobacterium spp., Haemophilus ducreyi, Haemophilus influenzae, Haemophilus parainfluenzae, Helicobacter pylori, Kingella kingae, Klebsiella oxytoca, Klebsiella ozaenae, Klebsiella pneumoniae, Klebsiella rhinoscleromatis, Legionella pneumophila, Moraxella catarrhalis, Morganella morganii, Neisseria gonorrhoeae, Neisseria meningitides, Pasteurella multocida, Plesiomonas shigelloides, Porphyromonas asaccharolytica, Porphyromonas gingivalis, Prevotella bivia, Prevotella buccae, Prevotella corporis, Prevotella endodontalis, Prevotella intermedia, Prevotella melaninogenica, Prevotella oralis, Proteus mirabilis, Proteus myxofaciens, Proteus penner, Proteus vulgaris, Providencia alcalifaciens, Providencia rettgeri, Providencia stuarfii, Pseudomonas aeruginosa, Pseudomonas fluorescens, Ricketsia prowozekii, Salmonella enterica, Serratia marcescens, Shigella boydii, Shigella dysenteriae, Shigella flexneri, Shigella sonnei, Stenotrophomonas maltophilia, Streptobacillus moniliformis, Vibrio alginolyticus, Vibrio cholerae, Vibrio parahaemolyticus, Vibrio vuluificus, Yersinia enterocolitica, Yersinia pestis* et *Yersinia pseudotuberculosis.*

**19.** Utilisation d’un composé selon l’une quelconque des revendications 1 à 10, ou d’un sel pharmaceutiquement acceptable, d’un solvate ou d’un ester de celui-ci, pour la fabrication d’un médicament pour le traitement d’un état tel que défini dans l’une quelconque des revendications 15 à 18.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5925659 A
- WO 200400744 A
- WO 2004062601 A
- WO 2007064732 A
- WO 2008027466 A
- JP 2002088046 A
- WO 9941276 A
- WO 9941246 A
- US 4256108 A
- US 4166452 A
- US 4265874 A


### Non-patent literature cited in the description


- **T. W. GREENE et al.** Protective Groups in organic Synthesis. Wiley, 1991
- Handbook of Pharmaceutical Salts. Properties, Selection and Use. Wiley-VCH, 2002
- **S. BERGE et al.** *Journal of Pharmaceutical Sciences,* 1977, vol. 66 (1), 1-19
- **P. GOULD.** *International J. of Pharmaceutics,* 1986, vol. 33, 201-217
- **ANDERSON et al.** The Practice of Medicinal Chemistry. Academic Press, 1996
- The Orange Book. Food & Drug Administration